# EUROPEAN PATENT SPECIFICATION

(11) **EP 3 473 294 B1**
(45) Date of publication and mention of the grant of the patent: **10.03.2021**
(21) Application number: 18211413.2
(22) Date of filing: 12.03.2014
(51) Int. Cl.: A61N 1/05, A61N 1/36, A61N 1/375, A61H 39/00, A61N 1/372, A61N 1/378

(54) **IMPLANTABLE ELECTROACUPUNCTURE DEVICE EMPLOYING A HIGH IMPEDANCE COIN-CELL BATTERY AS A PRIMARY POWER SOURCE**
IMPLANTIERBARE ELEKTROAKUPUNKTURVORRICHTUNG MIT KNOPFZELLE MIT HOHER IMPEDANZ ALS PRIMÄRSTROMQUELLE
DISPOSITIF D'ÉLECTROACUPUNCTURE IMPLANTABLE EMPLOYANT UNE PILE BOUTON À HAUTE IMPÉDANCE COMME SOURCE D'ALIMENTATION PRIMAIRE

(30) Priority: 12.03.2013 US 201361778018 P; 12.03.2013 US 201361778078 P; 12.03.2013 US 201361778061 P
(43) Date of publication of application: 24.04.2019
(62) Divisional of application: 14723913.1
(73) Proprietor: Valencia Technologies Corporation, Valencia, California 91355 (US)
(72) Inventor: PETERSON, David K.L., Valencia, California 91355 (US); GREINER, Jeffrey H., Valencia, California 91355 (US); THENUWARA, Chuladatta, Valencia, California 91355 (US); GREINER, Stacy O., Valencia, California 91355 (US)
(74) Representative: Schwan Schorer & Partner mbB

(56) References cited:
- WO-A1-01/41869
- WO-A1-02/00294
- US-A1- 2003 195 585
- US-A1- 2008 091 255
- US-A1- 2009 192 555
- US-B1- 7 013 177

## Description

### BACKGROUND

Acupuncture has been practiced in Eastern civilizations (principally in China, but also in other Asian countries) for at least 2500 years. It is still practiced today throughout many parts of the world, including the United States and Europe. The history of acupuncture, and its potential applications, is well documented, *see, e.g.,* Cheung, et al., "The Mechanism of Acupuncture Therapy and Clinical Case Studies", (Taylor & Francis, publisher) (2001) ISBN 0-415-27254-8.

Acupuncture is an alternative medicine that treats patients by insertion and manipulation of needles in the body at selected points. The locations where the acupuncture needles are inserted are referred to herein as "acupuncture points" or simply just "acupoints". The location of acupoints in the human body has been developed over thousands of years of acupuncture practice, and maps showing the location of acupoints in the human body are readily available in acupuncture books or online. For example, see the reference book cited below in Paragraph **[0004].** Acupoints are typically identified by various letter/number combinations, e.g., L6, S37. The maps that show the location of the acupoints may also identify what condition, illness or deficiency the particular acupoint affects when manipulation of needles inserted at the acupoint is undertaken.

References to the acupoints in the literature are not always consistent with respect to the format of the letter/number combination. Some acupoints are identified by a name only, e.g., *Tongli.* The same acupoint may be identified by others by the name followed with a letter/number combination placed in parenthesis, e.g., *Tongli* (HT5). Alternatively, the acupoint may be identified by its letter/number combination followed by its name, e.g., HT5 (*Tongli*). The first letter typically refers to a body organ, or meridian, or other tissue location associated with, or affected by, that acupoint. However, usually only the letter is used in referring to the acupoint, but not always. Thus, for example, the acupoint BL23 is the same as acupoint Bladder 23 which is the same as BL-23 which is the same as BL 23 which is the same as *Shenshu.* For purposes of this patent application, unless specifically stated otherwise, all references to acupoints that use the same name, or the same first letter and the same number, and regardless of slight differences in second letters and formatting, are intended to refer to the same acupoint.

An excellent reference book that identifies all of the traditional acupoints within the human body is WHO STANDARD ACUPUNCTURE POINT LOCATIONS IN THE WESTERN PACIFIC REGION, published by the World Health Organization (WHO), Western Pacific Region, 2008 (updated and reprinted 2009), ISBN 978 92 9061 248 7 (hereafter *"*WHO Standard Acupuncture Point Locations 2008"). This reference book, WHO Standard Acupuncture Point Locations 2008*,* is incorporated herein by reference.

As an alternative to traditional acupuncture, some have proposed applying moderate electrical stimulation at selected acupuncture points through needles that have been inserted at those points. Such electrical stimulation is known as electroacupuncture (EA). Electroacupuncture is quite similar to traditional acupuncture in that the same points are stimulated during treatment. As with traditional acupuncture, when practicing electroacupuncture, needles are inserted on specific points along the body. The needles are then attached to a device that generates continuous electric pulses using small clips. These devices are used to adjust the frequency and intensity of the impulse being delivered, depending on the condition being treated. Electroacupuncture uses two needles at a time so that the impulses can pass from one needle to the other. Several pairs of needles can be stimulated simultaneously, usually for no more than 30 minutes at a time. It should be noted that other medical research, not associated with acupuncture research, has over the years identified nerves and other locations throughout a patient's body where the application of electrical stimulation produces a beneficial effect for the patient. Indeed, the entire field of neurostimulation deals with identifying locations in the body where electrical stimulation can be applied in order to provide a therapeutic effect for a patient. For purposes of this patent application, such known locations within the body are treated essentially the same as acupoints - they provide a "target" location where electrical stimulation may be applied to achieve a beneficial result, whether that beneficial result is to treat erectile dysfunction, reduce cholesterol or triglyceride levels, to treat cardiovascular disease, to treat mental illness, or to address some other issue associated with a disease or condition of the patient.

United States Patent 6,735,475, issued to Whitehurst et al., discloses use of an implantable miniature neurostimulator, referred to as a "microstimulator," that can be implanted into a desired tissue location and used as a therapy for headache and/or facial pain. The microstimulator has a tubular or cylindrical shape, with electrodes at each end.

Other patents of Whitehurst et al. teach the use of this small, microstimulator, placed in other body tissue locations, including within an opening extending through the skull into the brain, for the treatment of a wide variety of conditions, disorders and diseases. *See, e.g.,* US Patent 6,950,707 (obesity and eating disorders); US Patent 7,003,352 (epilepsy by brain stimulation); US Patent 7,013,177 (pain by brain stimulation); US Patent 7,155,279 (movement disorders through stimulation of Vagus nerve with both electrical stimulation and drugs); US Patent 7,292,890 (Vagus nerve stimulation); US Patent 7,203,548 (cavernous nerve stimulation); US Patent 7,440,806 (diabetes by brain stimulation); US Patent 7,610,100 (osteoarthritis); and US Patent 7,657,316 (headache by stimulating motor cortex of brain).

Techniques for using electrical devices, including external and implanted EA devices, for stimulating peripheral nerves and other body locations for treatment of various maladies are known in the art. See, e.g., U.S. Patent Nos. 4,535,784; 4,566,064; 5,195,517; 5,211,175; 5,250,068; 5,251,637; 5,891,181; 6,393,324; 6,006,134; 7,171,266; and 7,373,204. The methods and devices disclosed in these patents, however, typically utilize (i) large implantable stimulators having long leads that must be tunneled through tissue or pass through blood vessels over an extended distance to reach the desired stimulation site, (ii) external devices that must interface with implanted electrodes via percutaneous leads or wires passing through the skin, or (iii) inefficient and power-consuming wireless transmission schemes. Such devices and methods are still far too invasive, or are ineffective, and thus are subject to the same limitations and concerns, as are the previously described electrical stimulation devices.

From the above, it is seen that there yet remains a need in the art for a less invasive device and technique for electroacupuncture stimulation of acupoints, or other target tissue locations within the body, that does not require the continual use of needles inserted through the skin, or long insulated wires implanted or inserted into blood vessels, for the purpose of treating an illness or deficiency of a patient. Likewise, there is also a need for a new type of packaging associated with an implantable electroacupuncture device, including the ability to safely use radial feed-through pins therewith.

### BRIEF DESCRIPTION OF THE DRAWINGS

The accompanying drawings illustrate various embodiments of the principles described herein and are part of the specification. The illustrated embodiments are merely examples and do not limit the scope of the disclosure. Throughout the drawings, identical or similar reference numbers designate identical or similar elements.
FIG. 1 is a perspective view of an Implantable Electroacupuncture Device (IEAD) made in accordance with the teachings presented herein.
FIG. 1A illustrates the location of an exemplary target tissue stimulation site, e.g., an acupoint, and an IEAD of the type shown in FIG. 1 implanted so as to underlie such target tissue location.
FIG. 1B shows a sectional view of an IEAD implanted at a selected target stimulation site, and illustrates the electric field gradient lines created when an electroacupuncture (EA) pulse is applied to the tissue through the central electrode and ring electrode attached to the bottom surface and perimeter edge, respectively, of the IEAD housing.
FIG. 2 shows a plan view of one surface (identified in FIG. 2 as the "Cathode Side") of the IEAD housing illustrated in FIG.1.
FIG. 2A shows a side view of the IEAD housing illustrated in FIG. 1.
FIG. 3 shows a plan view of the other side, indicated as the "Skin Side," of the IEAD housing or case illustrated in FIG. 1.
FIG. 3A is a sectional view of the IEAD of FIG. 3 taken along the line A-A of FIG. 3.
FIG. 4 is a perspective view of the IEAD housing, including a feed-through pin, before the electronic components are placed therein, and before being sealed with a cover plate.
FIG. 4A is a side view of the IEAD housing of FIG. 4.
FIG. 5 is a plan view of the empty IEAD housing shown in FIG. 4.
FIG. 5A depicts a sectional view of the IEAD housing of FIG. 5 taken along the section line A-A of FIG. 5.
FIG. 5B shows an enlarged view or detail of the portion of FIG. 5A that is encircled with the line B.
FIG. 5C is a plan view similar to FIG. 5 showing the use of multiple recess cavities in the IEAD housing, each with its own respective radial feed-through pin passing through the bottom of its recess cavity.
FIG. 5D is a plan view similar to FIG. 5 showing the use of multiple radial feed-through pins passing through the bottom of a single recess cavity.
FIG. 6 is a perspective view of an electronic assembly, including a battery, adapted to fit inside of the empty housing of FIG. 4 and FIG. 5.
FIG. 6A and 6B show a plan view and side view, respectively, of the electronic assembly shown in FIG. 6.
FIG. 7 is an exploded view of the IEAD assembly, illustrating its constituent parts.
FIG. 7A schematically illustrates a few alternative electrode configurations that may be used with the invention.
FIG. 8 illustrates a functional block diagram of the electronic circuits used within an IEAD of the type described herein.
FIG. 9 shows a basic boost converter circuit configuration, and is used to model how the impedance of the battery R_{BAT} can affect its performance.
FIG. 9A illustrates a typical voltage and current waveform for the circuit of FIG. 8 when the battery impedance R_{BAT} is small.
FIG. 9B shows the voltage and current waveform for the circuit of FIG. 8B when the battery impedance R_{BAT} is large.
FIG. 10 shows one preferred boost converter circuit and a functional pulse generation circuit configuration for use within the IEAD.
FIG. 11 shows an alternate boost converter circuit configuration and a functional pulse generation circuit for use within the IEAD.
FIG. 12 shows a refinement of the circuit configuration of FIG. 11.
FIG. 13A shows one preferred schematic configuration for an implantable IEAD that utilizes the boost converter configuration shown in FIG. 10.
FIG. 13B shows current and voltage waveforms associated with the operation of the circuit shown in FIG. 13A.
FIG. 14 shows another preferred schematic configuration for an IEAD similar to that shown in FIG. 13A, but which uses an alternate output circuitry configuration for generating the stimulus pulses.
FIG. 14A depicts yet a further preferred schematic configuration for an IEAD similar to that shown in FIG. 13A or FIG. 14, but which includes additional enhancements and circuit features.
FIGS. 14B and 14C show timing waveform diagrams that illustrate the operation of the circuit of FIG. 14 before (FIG. 14B) and after (FIG. 14C) the addition of a cascode stage to the IEAD circuitry that removes some undesirable transients from the leading edge of the stimulus pulse.
FIGS. 14D and 14E illustrate timing waveform diagrams that show the operation of the circuit of FIG. 14 before (FIG. 14D) and after (FIG. 14E) the addition of circuitry that addresses a delay when starting the current regulator U3 for low amplitude stimulus pulses.
FIG. 15 shows a reverse trapezoidal waveform of the type that is generated by the pulse generation circuitry of the IEAD, and further illustrates one approach for achieving the desired reverse trapezoidal waveform shape.
FIG. 15A shows a timing waveform diagram of representative EA stimulation pulses generated by the IEAD device during a stimulation session.
FIG. 15B shows a timing waveform diagram of multiple stimulation sessions, and illustrates the waveforms on a more condensed time scale.
FIG. 15C illustrates another preferred schematic configuration for an IEAD similar to that shown in FIG. 13A, FIG. 14 or FIG. 14A, but which includes a real time clock module to better facilitate chronotherapeutic applications of the stimulation regimen.
FIG. 16 shows a state diagram that depicts the various states the IEAD may assume as controlled by an external magnet.
FIG. 17 illustrates various exemplary acupoints on a patient's body.
FIG. 17A shows a sectional view of a first way that an IEAD may be implanted at a selected target stimulation site when there is a bone or other skeletal structure that prevents the IEAD from being implanted very deep below the skin.
FIG. 17B shows a sectional view of a second way that an IEAD may be implanted at a selected target stimulation site when there is a bone or other skeletal structure that prevents the IEAD from being implanted very deep below the skin.

### DETAILED DESCRIPTION

The invention relates to an implantable electroacupuncture device as defined in claims 1 and 8, respectively. Described herein is an implantable, self-contained, electroacupuncture (EA) device that is powered by a small, thin, coin-cell type battery having an internal impedance of at least five ohms (Ω). While the device described is intended for, and is described for use as, an EA device, it should be noted that it may be used for other similar tissue stimulation applications. In an exemplary embodiment, the EA device includes two electrode contacts mounted on or connected to the surface of its housing. The EA device is adapted to treat a particular disease or health condition of a patient. In one embodiment, the electrodes include a central cathode electrode on one side of the housing, and an annular anode electrode that surrounds the cathode. In another embodiment, the anode annular electrode is a ring electrode placed around the perimeter edge of a coin-shaped housing.

As indicated above, the preferred EA device is leadless. This means there are no leads or electrodes at the distal end of leads (common with most implantable electrical stimulators) that have to be positioned and anchored at a desired stimulation site. Also, because there are no leads, no tunneling through body tissue or blood vessels is required in order to provide a path for the leads to return and be connected to a tissue stimulator (also common with most electrical stimulators).

The EA device is adapted to be implanted through a very small incision, e.g., less than 2-3 cm in length, directly adjacent to a selected target stimulation site, e.g., an acupoint, known to moderate or affect an identified health condition of a patient.

The EA device is easy to implant. Also, most embodiments are symmetrical. This means that there is no way that it can be implanted incorrectly (unless the physician puts it in upside-down, which would be difficult to do given the markings on its case). All that need be done is to cut the incision, and slide the device in place through the incision. Once the implant pocket has been prepared, it is as easy as sliding a coin into a slot. Such implantation can usually be completed in less than 10 minutes in an outpatient setting, or in a doctor's office. Only minor, local anesthesia need be used. No major or significant complications are envisioned for the implant procedure. The EA device can also be easily and quickly explanted, if needed or desired.

The EA device is self-contained. It includes a primary battery to provide its operating power. Such primary battery has a high impedance, greater than 5 ohms. In view of such high impedance, the EA device includes battery control circuitry that limits the amount of instantaneous current drawn from the primary battery to prevent excessive voltage drops in the output voltage of the battery. Such battery control circuitry carefully manages the delivery of power by the EA device so as to allow the device to perform its intended function for several years.

Once the EA device is implanted in a patient, the patient should not even know it is there, except for a slight tingling that may be felt when the device is delivering bursts of stimulus pulses during a stimulation session. Also, once implanted, the patient can just forget about it. There are no complicated user instructions that must be followed. Just turn it on. No maintenance is needed. Moreover, should the patient want to disable the EA device, i.e., turn it OFF, or change stimulus intensity, he or she can do so using, e.g., an external magnet.

The EA device can operate for several years because it is designed to be very efficient. Stimulation pulses applied by the EA device at a selected target stimulation site, e.g., a specified acupoint, are applied at a very low duty cycle in accordance with a specified stimulation regimen. The stimulation regimen applies continuous EA stimulation pulses during a stimulation session that lasts at least 10 minutes, typically 30 minutes, and rarely longer than 60 minutes. These stimulation sessions, however, occur at a very low duty cycle. In one preferred treatment regimen, for example, a stimulation session having a duration of 30 minutes is applied to the patient just once a week. The stimulation regimen, and the selected target tissue location, e.g., acupoint, at which the stimulation is applied, are designed and selected to provide efficient and effective EA stimulation for the treatment of the patient's medical condition.

The EA device is, compared to most implantable medical devices, relatively easy to manufacture and uses few components. This not only enhances the reliability of the device, but keeps the manufacturing costs low, which in turn allows the device to be more affordable for the patient.

In operation, the EA device is safe to use. There are no horrific failure modes that could occur. Because it operates at a very low duty cycle (i.e., it is OFF much, much more than it is ON), it generates little heat. Even when ON, the amount of heat it generates is not much, less than 1 mW, and is readily dissipated. Should a component or circuit inside of the EA device fail, the device will simply stop working. If needed, the EA device can then be easily explanted.

A key feature included in the design of the EA device is the use of a commercially-available battery as its primary power source. A preferred commercially-available battery to use in the EA device is a small, thin, disc-shaped battery, also known as a "coin cell" battery, such as the 3 V CR1612 lithium battery available from Panasonic, or equivalents thereof. Such coin-cell batteries are quite common and readily available for use with most modem hand-held electronic devices. Such batteries come in many sizes, and employ various configurations and materials. However, insofar as the inventors or Applicant are aware, such batteries have never been used in implantable medical devices previously. This is because their internal impedance is, or has always thought to have been, much too high for such batteries to be of practical use within an implantable medical device where power consumption must be carefully monitored and managed so that the device's battery will last as long as possible. Further, because of the high internal impedance, dips in the battery output voltage (caused by any sudden surge in instantaneous battery current) may occur that could compromise the performance of the device. Additionally, the energy requirements of other active implantable therapies are far greater than can be provided by such coin cells without frequent replacement.

The EA device disclosed herein advantageously employs power-monitoring and power-managing circuits that prevent any sudden surges in battery instantaneous current, or the resulting drops in battery output voltage, from ever occurring, thereby allowing a whole family of commercially-available, very thin, high-output-impedance, relatively low capacity, small disc batteries (or "coin cells") to be used as the EA device's primary battery without compromising the EA device's performance. As a result, instead of specifying that the EA device's battery must have a high capacity, e.g., greater than 200 mAh, with an internal impedance of, e.g., less than 5 ohms, which would either require a thicker battery and/or preclude the use of commercially-available coin-cell batteries, the EA device of the present invention can readily employ a battery having a relatively low capacity, e.g., less than 60 mAh, and a high battery impedance, e.g., greater than 5 ohms, and typically over 100 ohms.

Moreover, the power-monitoring, power-managing, as well as the pulse generation, and control circuits used within the EA device are relatively simple in design, and may be readily fashioned from commercially-available integrated circuits (IC's) or application-specific integrated circuits (ASIC's), supplemented with discrete components, as needed. In other words, the electronic circuits employed within the EA device need not be complex nor expensive, but are simple and inexpensive, thereby making it easier to manufacture and to provide the device to patients at an affordable cost.

Additionally, in a coin-sized implantable EA device of the type disclosed herein, a feed-through connection is required to electrically connect the output of the electronic circuitry located inside of the hermetically-sealed, thin profile implant case (which is approximately only about 2.5 mm thick) to a ring-shaped anode electrode mounted on the cylindrical surface of the perimeter of the case.

More particularly, as is evident from the figures, and their accompanying descriptions presented herein, the feed-through assembly design described herein incorporates a radial recess at the edge of the IEAD package where the feed-through assembly is placed. The distal tip of the feed-through pin advantageously extends beyond the edge of the package where it can readily be attached to a ring electrode that fits over the edge of the package. Yet, the actual feed-through assembly, i.e., that more proximal portion of the feed-through pin or wire that is brazed and embedded within a ruby bead, or other insulating material, is in the bottom of the recess, and therefore sufficiently thermally and mechanically far away from the high temperatures associated with a laser seam weld when the welding is performed.

It is noted that some of the meticulous details associated with the manufacture and assembly of the EA device described herein are not provided. This is because such details should be known to a person of ordinary skill in the art. In particular, in combination with the teachings presented herein, the person of skill in the art should be able to discern how the feed-through assembly described herein is manufactured, and how such assembly is then incorporated into the overall hermetically-sealed package design of the EA device described.

As used herein, "annular", "circumferential", "circumscribing", "surrounding" or similar terms used to describe an electrode or electrode array, or electrodes or electrode arrays, (where the phrase "electrode or electrode array," or "electrodes or electrode arrays," is also referred to herein as "electrode/array," or "electrodes/arrays," respectively) refers to an electrode/array shape or configuration that surrounds or encompasses a point or object, such as another electrode, without limiting the shape of the electrode/array or electrodes/arrays to be circular or round. In other words, an "annular" electrode/array (or a "circumferential" electrode/array, or a "circumscribing" electrode/array, or a "surrounding" electrode/array), as used herein, may be many shapes, such as oval, polygonal, starry, wavy, and the like, including round or circular.

"Nominal" or "about" when used with a mechanical dimension, e.g., a nominal diameter of 23 mm, means that there is a tolerance associated with that dimension of no more than plus or minus (+/-) 5%. Thus, a dimension that is nominally 23 mm means a dimension of 23 mm +/-1.15 mm (0.05x23 mm=1.15 mm).

"Nominal" when used to specify a battery voltage is the voltage by which the battery is specified and sold. It is the voltage you expect to get from the battery under typical conditions, and it is based on the battery cell's chemistry. Most fresh batteries will produce a voltage slightly more than their nominal voltage. For example, a new nominal 3 volt lithium coin-sized battery will measure more than 3.0 volts, e.g., up to 3.6 volts under the right conditions. Since temperature affects chemical reactions, a fresh warm battery will have a greater maximum voltage than a cold one. For example, as used herein, a "nominal 3 volt" battery voltage is a voltage that may be as high as 3.6 volts when the battery is brand new, but is typically between 2.7 volts and 3.4 volts, depending upon the load applied to the battery (i.e., how much current is being drawn from the battery) when the measurement is made and how long the battery has been in use.

As explained in more detail below, an important aspect associated with the use of the EA device described herein recognizes that an electroacupuncture (EA) modulation scheme, or other tissue stimulation scheme or regimen, need not be continuous, thereby allowing the implanted device to use a small, high density, power source to provide such non-continuous modulation. (Here, it should be noted that "modulation," as that phrase is used herein, is the application of electrical stimulation pulses, at low intensities, low frequencies and low duty cycles, to at least one of the target stimulation sites, e.g., an acupuncture site, that has been identified as affecting a particular condition of a patient.) As a result, the device can be very small. And, because the electrodes typically form an integral part of the housing of the device, the device may thus be implanted directly at (or very near to) the desired target tissue location.

As described in more detail below, the basic approach of EA stimulation disclosed herein includes: (1) identify an acupoint(s) or other target stimulation site that may be used to treat or mediate a particular illness, condition or deficiency that has manifest itself in a patient, e.g., high blood pressure; (2) implant an EA device, made as described herein, so that its electrodes are located to be near or at the identified acupoint(s) or other target stimulation site; (3) apply EA modulation, having a low intensity, low frequency, and low duty cycle through the electrode(s) of the EA device so that electrical stimulation pulses flow through the tissue at the target stimulation site following a prescribed stimulation regimen over several weeks or months or years. At any time during this EA stimulation regimen, the patient's illness, condition or deficiency may be evaluated and, as necessary, the parameters of the EA modulation applied during the EA stimulation regimen may be adjusted or "tweaked" in order to improve the results obtained from the EA modulation.

The IEAD disclosed herein may be used to treat many different medical conditions of a patient, including, *inter alia,* (1) hypertension, (2) cardiovascular disease, (3) depression, (4) bipolar disorder, (5) Anxiety, (6) obesity, (7) dyslipidemia, (8) Parkinson's disease, (9) Essential tremor, and (10) erectile dysfunction (ED). For each of these ten enumerated conditions, Applicant has performed extensive research in the acupuncture art to determine which acupoints are likely to be the best candidates for being modulated (i.e., stimulated with electroacupuncture pulses) in order to treat and/or provide some meaningful relief relative to symptoms of these conditions. As a result of this research, Applicant has identified at least one acupoint for each of the above-identified ten enumerated conditions/diseases, which at least one acupoint represents -insofar as the inventors are presently aware- the best candidate(s) for where EA stimulation from an IEAD of the type described herein should be applied for successful treatment of the condition/disease. Some overlap exists between the identified acupoints, i.e., in some instances the same acupoint(s) may be stimulated to treat multiple conditions. The results of this research are summarized in Table 1.

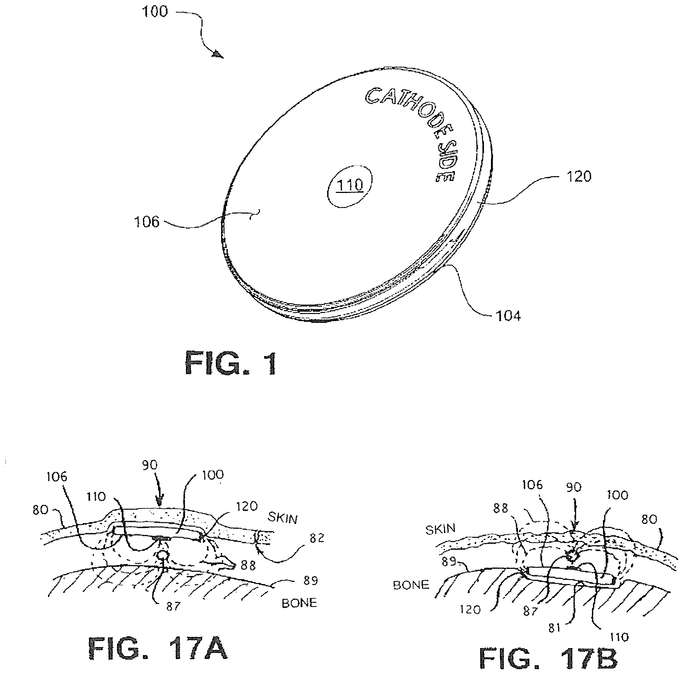

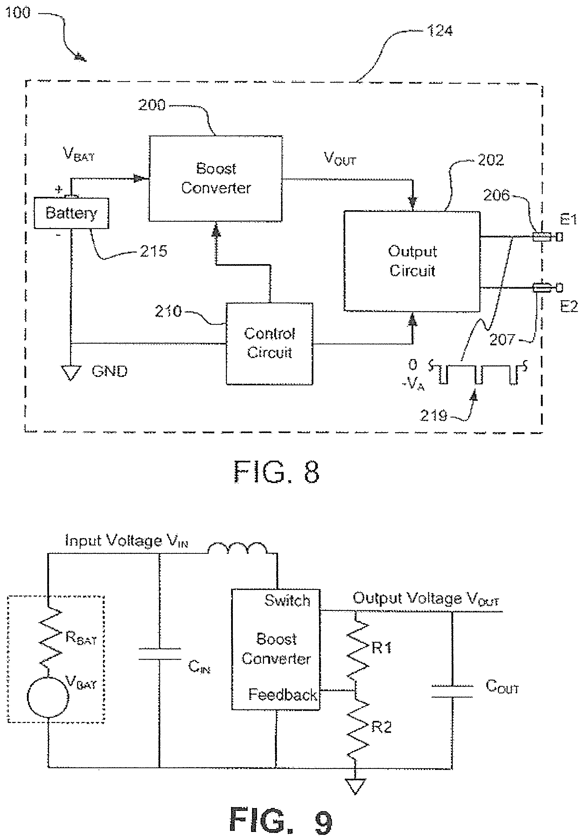

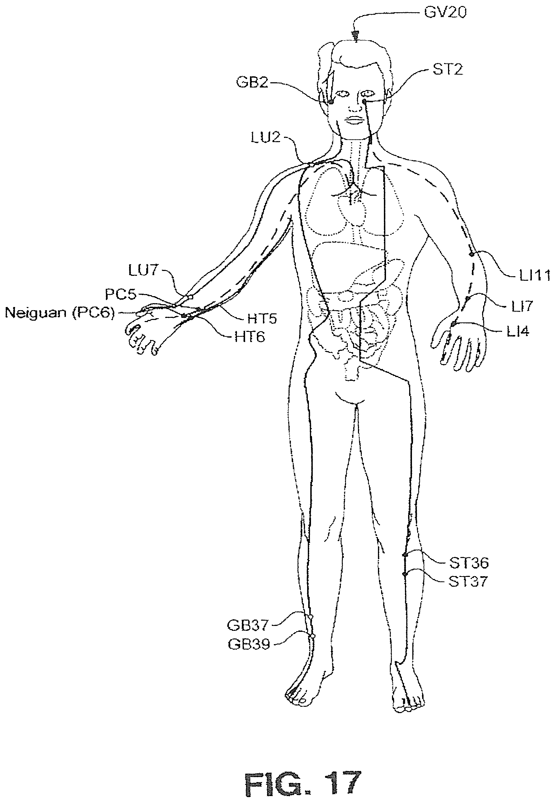

As seen in Table 1, the ten conditions are listed in the left column. The acupoint(s) that represents, based on Applicant's research, the best candidate(s) for applying electrical stimulation pulses in order to treat the indicated condition, is/are listed in the column of Table 1 labeled "Acupoints (Target Tissue Locations)". The nomenclature used in the reference book WHO Standard Acupuncture Point Locations 2008*,* see Paragraph **[0004]** above, is used to identify these acupoints. FIG. 17 also shows the location of many of these acupoints. Finally, in the six columns on the right of Table 1, the stimulation parameters thought by the inventors at the present time to provide the most effective stimulation regimen for the identified acupoint(s) are set forth. The meaning of these parameters will become apparent from the description that follows, as well as the description of the IEAD presented below in connection with the description of, e.g., FIGS. 8A, 10, 14A, 15A and 15B.

It should It be noted that the ten conditions enumerated in Table 1 are only exemplary of a small set of conditions of what Applicant believes may eventually be a much larger set of conditions that can be treated by using an IEAD as described herein to apply electrical stimulation to selected target tissue locations throughout a patient's body.

**TABLE 1. Conditions Treated through indicated Target Tissue Locations and Stimulation Regimen Parameters****

| **Condition or Illness Treated** | **Acupoint(s)*** (Target Tissue Locations) | **T3** (min) | **T4** (days) | **T1** (msec) | **T2** (msec) | **A1** (volts) | **A1** (mA) |
|---|---|---|---|---|---|---|---|
| Hypertension | **PC5, PC6, ST36, ST37,** LI4, LI11, LR3, GB34 | 10 - 60 | ½ - 14 | 0.1 - 2.0 | 200 - 2000 | 1 - 15 | 1 - 25 |
| Card iovascular Disease | **PC6** (or on an axis line connecting PC6 with either PC5 or PC7), ST36, BL14, HT7, EXHN1, HT5, LI11, LU2, LU7 (or underlying nerves) | 10 - 60 | ½ -14 | 0.1 - 1.0 | 250 - 1000 | 1 - 15 | 1 - 25 |
| Depression | **GV20, EXHN3** or underlying nerves (e.g., trigeminal nerve or three branches of trigeminal nerve) | 10 - 70 | 1 - 14 | 0.1 - 1.0 | 100 - 1000 | 1 -15 | 1 - 25 |
| Bipolar Disorder | **GV20, EXHN3** or underlying nerves | 10 - 70 | 1 - 14 | 0.1 - 1.0 | 250 - 1000 | 1 - 15 | 1 - 25 |
| Anxiety | **GV20, EXHN3** or underlying nerves | 10 - 70 | ½ - 14 | 0.1 - 1.0 | 100 - 1000 | 1 - 15 | 1 - 25 |
| Obesity | **SP4, LR8, ST40,** ST36, ST37, SP6, SP9, KI6 | 20 - 60 | ½ - 14 | 0.1 - 1.0 | 250 - 1000 | 1 - 15 | 1 - 25 |
| Dyslipidemia | **ST40,** ST36, SP4, ST37, LR8, SP6, SP9, KI6 | 20 - 60 | 1 - 14 | 0.1 - 1.0 | 250 - 1000 | 1 - 15 | 1 - 25 |
| Parkinson's Disease | **GV20, GB34** or underlying nerves | 10 - 60 | 1 - 14 | 0.1 - 2.0 | 67 - 1000 | 1 - 15 | 1 - 25 |
| Essential Tremor | **GV20, GB34** or underlying nerves | 10 - 60 | 1 - 14 | 0.1 - 2.0 | 67 - 1000 | 1 - 15 | 1 - 25 |
| Erectile Dysfunction | **BL52, BL23, GV4** | 20 - 60 | 1 - 14 | 0.1 - 2.0 | 67 - 1000 | 1 - 20 | 1 - 25 |

| | | | | | | | |
|---|---|---|---|---|---|---|---|
| *Bold font indicates a preferred target ** Duty Cycle of T3/T4 ≤ 0.05 prevents long values of T3 and/or short values of T4 in some instances | | | | | | | |

The list of conditions presented in Table 1 is an "open" list, not a "closed" list. The inventors of the EA device described herein believe that this device, or equivalents thereof, will prove to be a useful tool not only to provide needed treatment and relief for patients suffering from any of these enumerated conditions, but will also promote additional research that will identify many more conditions and associated target tissue locations whereat EA stimulation can be successfully applied.

In this regard, it should further be emphasized that "EA stimulation" or "EA modulation", as those terms are used herein, is not intended to limit the resulting electrical stimulation pulses to be applied at a specified acupoint. Rather, for purposes herein, "EA stimulation" or "EA modulation" is electrical stimulation that is applied to whatever target tissue stimulation point has been selected in a manner consistent with the teachings herein, i.e., at a very low duty cycle. This low duty cycle (less than 0.05) means using stimulation sessions that have a duration of 10-60 minutes, and where the stimulation sessions are applied no more than once a day, but usually only once a week or once every other week. Further, "EA stimulation" or "EA modulation" means, most of the time, using stimulation pulses during a stimulation session that are at a low frequency (e.g., 1 Hz to 15 Hz), low intensity (less than 25 mA amplitude), and narrow pulse width (e.g., from 0.1 to 2 m). Finally, "EA stimulation" or "EA modulation" means applying the electrical stimulation through electrodes that are either attached directly to the case of the EA device, or coupled to the EA device, i.e., the IEAD, through a very short lead. In other words, there are no needles inserted through the skin to reach the target stimulation site, as occurs in traditional acupuncture, or traditional electroacupuncture. Rather, everything associated with the applied EA stimulation is done with a self-contained EA device, and its electrodes, that is implanted.

Table 1 defines the stimulation parameters associated with the EA stimulation that should be applied to at least one of the indicated acupoints or other target tissue locations for treatment of the specified condition also shown or listed in Table 1. In general, these parameters are typical of low-frequency EA stimulation. That is, the EA stimulation should be applied at a low frequency, low intensity, and narrow pulse width. The pulse width of the stimulation pulse is defined as a time T1. The time interval between the start of one stimulation pulse and the start of the next stimulation pulse is a time T2. The frequency, or rate of occurrence of the stimulation pulses is thus 1/T2, expressed in units of pulses/sec, or Hz. The ratio of T1/T2 should be no greater than about 0.03, and is usually less than 0.01, thus assuring a narrow stimulation pulse width. The intensity, or amplitude, of the stimulation pulses (measured in either voltage or current, i.e., units of volts or milliAmps, or mA) is defined as A1. If the stimulation session has a duration of T3 minutes, and the stimulation sessions occur only once every T4 minutes (which may be expressed in units of minutes, hours, days or weeks, but care must be exercised when determining the duty cycle, or T3/T4, to ensure that the same units are used for both T3 and T4), then the duty cycle is T3/T4. In accordance with the operation restrictions of Applicant's IEAD, the duty cycle should be (must be) no greater than 0.05 in order to preserve the stored power of the small, thin, primary battery carried inside of the IEAD, thus allowing the IEAD to operate for several years.

In summary, it is to be emphasized that the duration and rate of occurrence of the EA stimulation pulses applied by the IEAD described herein are not arbitrary nor chosen haphazardly or by guesswork. Rather these parameters have been chosen after a careful examination of the reports of successful manual acupuncture studies.

As can be seen from Table 1, the duration T3 of the stimulation sessions for the ten conditions listed in Table 1 varies from as short as ten minutes to as long as about 70 minutes; the interval between stimulation sessions T4 varies from as short as ½ day to as long as 14 days. The stimulus pulses during a stimulation session have a pulse width T1 that is, in some instances, as short as 0.1 milliseconds (ms) and, in other instances, as long as 2 ms. The period between application of stimulus pulses varies from as short as 67 milliseconds (ms), corresponding to a rate of about 15 Hz, to as long as 1 second, corresponding to a rate of 1 Hz. The amplitude A1 of the stimulus pulses varies from as low as 1 mA to as much as 25 mA. The parameters T1, T2, T3, T4 and A1 define the stimulation paradigm or regimen that is applied by the IEAD at a selected target tissue location.

A representative pulse width of the stimulus pulse T1 is 0.5 ms. A representative period T2 for the stimulus pulse rate is 500 ms = 0.5 seconds (rate = 1/0.5 = 2Hz). A representative duration of a stimulation session T3 is thirty minutes, and a representative rate of occurrence of the stimulation session T4 is once every week. (Note: duty cycle T3/T4 = 30 min/10,080 min = 0.003.) A representative amplitude A1 of the stimulus pulse is, e.g., 15 mA, but can be set as high as 25 mA.

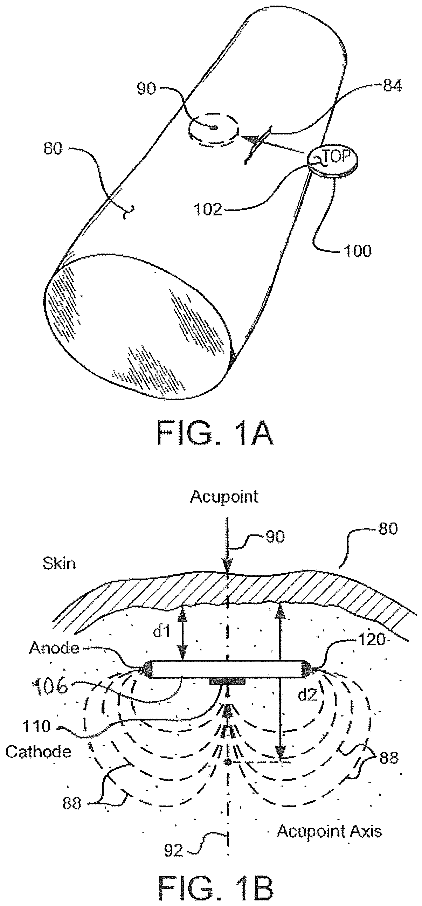

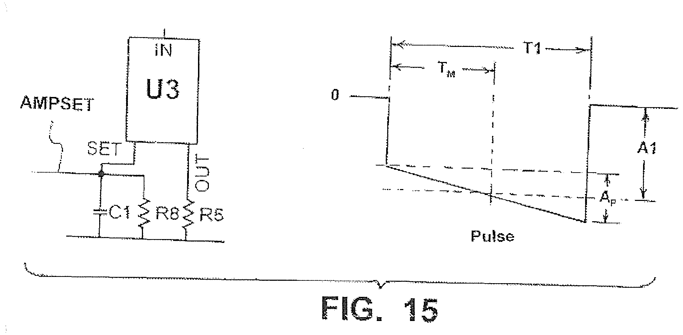

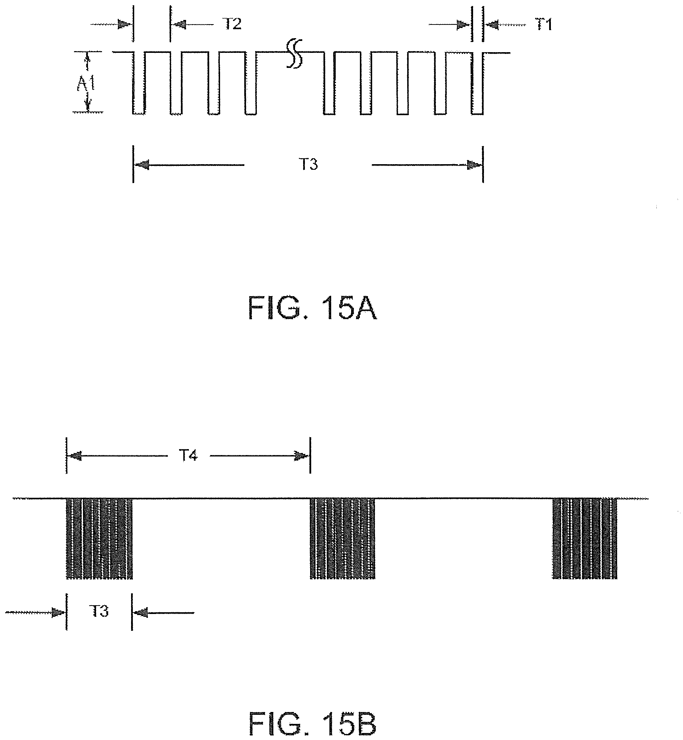

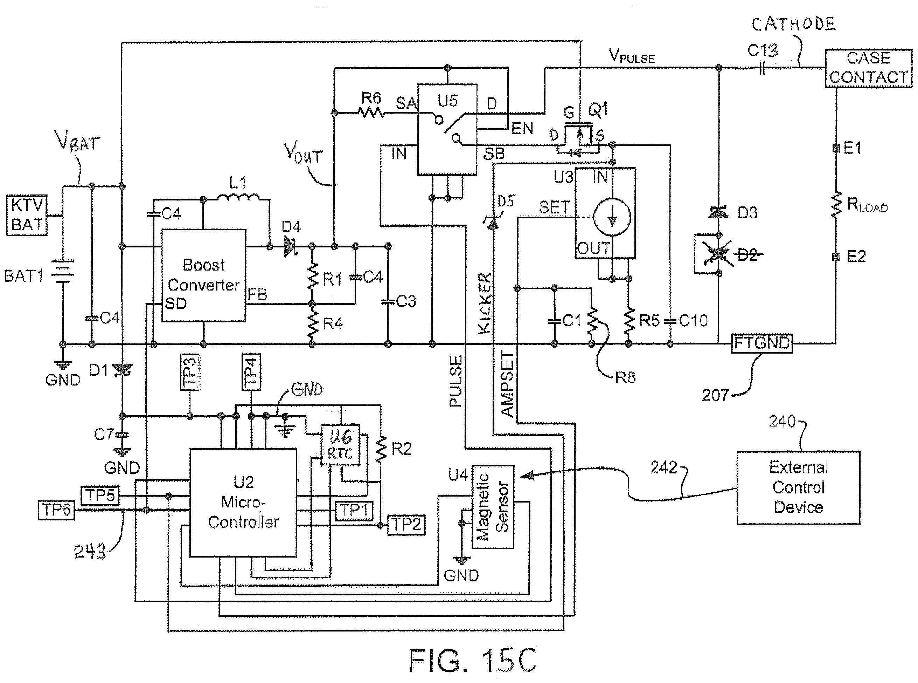

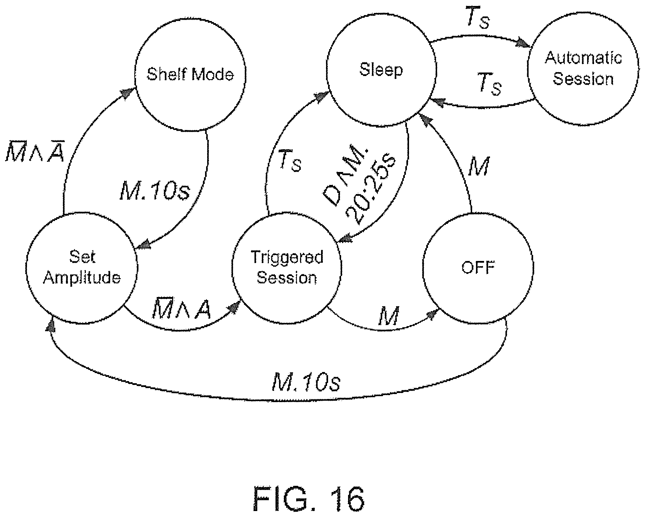

The mechanical design and implantation of an implantable electroacupuncture device (IEAD) will next be described with reference to FIGS. 1 through 7A, and FIGS. 17A through FIG 18. Following this description, the IEAD will be described electrically with reference to FIGS. 8 through 15, and FIG. 15C. Lastly, the use and operation of the device will be described with reference to FIGS. 15A, 15B and 16.

Turning to FIG. 1, a preferred configuration of a small IEAD 100 is shown in perspective view. (Note, throughout the description herein the IEAD 100 may also be referred to as an EA device. Likewise, the IEAD 100, or EA device, may sometimes be referred to as an implantable electroacupuncture stimulator (IEAS).) The IEAD is designed to be used, when implanted within a patient, to treat a disease, deficiency, or other medical condition of the patient. As seen in FIG. 1, the IEAD 100 has the appearance of a disc or coin, having: (a) a front side 106 (which is also labeled as the "Cathode Side"); (b) a back side 102 (also referred to as the "Skin Side", but which side 102 is not visible in FIG. 1); and (c) an edge side 104.

As used herein, the "front" side 106 of the IEAD 100 is the side that is positioned so as to face the target stimulation point (e.g., the target tissue location, such as a desired acupoint) where EA stimulation is to be applied when the IEAD is implanted. The "back" side 102 is the side opposite the front side and is the farthest away from the target stimulation point when the IEAD is implanted, and is usually the side closest to the patient's skin. The "edge" side 104 of the IEAD is the side that connects or joins the front side 106 to the back side 102. In FIG. 1, the IEAD 100 is oriented to show the front side 102 and a portion of the edge side 104.

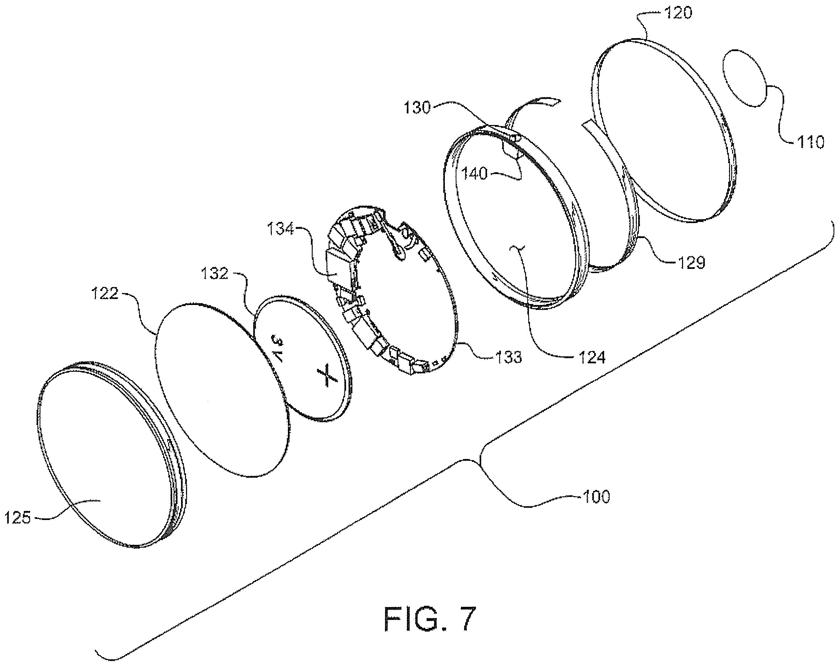

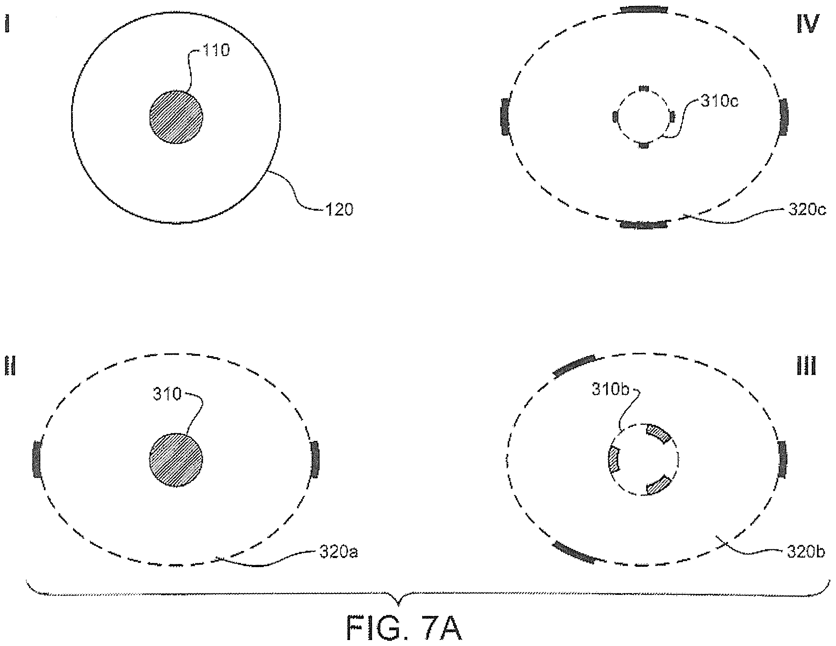

It should be noted here that throughout this application, the terms IEAD 100, IEAD housing 100, bottom case 124, can 124, or IEAD case 124, or similar terms, are used to describe the housing structure of the EA device. In some instances it may appear as if these terms are used interchangeably. However, the context should dictate what is meant by these terms. As the drawings illustrate, particularly FIG. 7, there is a bottom case 124 that comprises the "can" or "container" wherein the components of the IEAD 100 are first placed and assembled during manufacture of the IEAD 100. When all of the components are assembled and placed within the bottom case 124, a cover plate 122 is welded to the bottom case 124 to form an hermetically-sealed housing of the IEAD. The cathode electrode 110 is attached to the outside of the bottom case 124 (which is the front side 106 of the device), and the ring anode electrode 120 is attached, along with its insulating layer 129, around the perimeter edge 104 of the bottom case 124. Finally, a layer of silicone molding 125 covers the IEAD housing except for the outside surfaces of the anode ring electrode and the cathode electrode.

The embodiment of the IEAD 100 shown in FIG. 1 utilizes two electrodes, a cathode electrode 110 that is centrally positioned on the front side 106 of the IEAD 100, and an anode electrode 120. The anode electrode 120 is a ring electrode that fits around the perimeter edge 104 of the IEAD 100. Not visible in FIG. 1, but which is described hereinafter in connection with the description of FIG. 7, is a layer of insulating material 129 that electrically insulates the anode ring electrode 120 from the perimeter edge 104 of the housing or case 124.

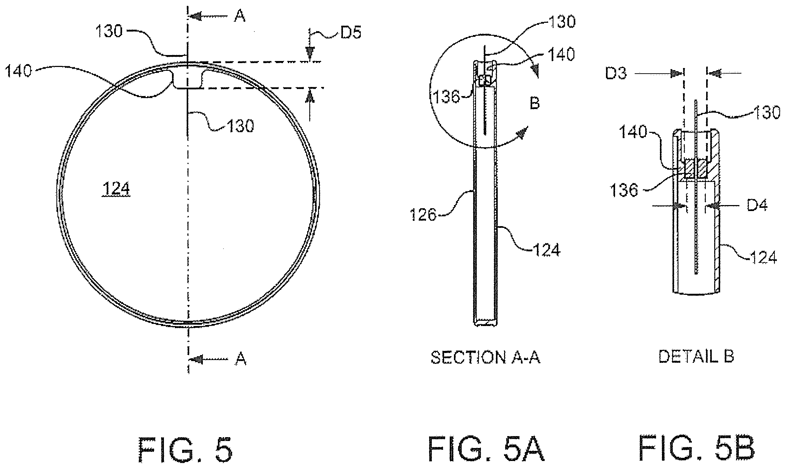

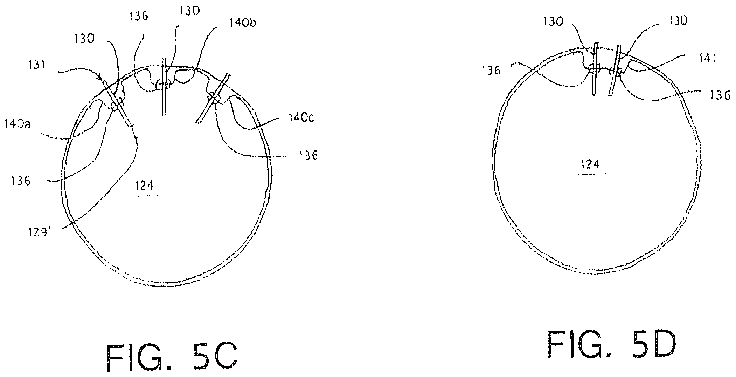

Also not visible in FIG. 1, but a key feature of the mechanical design of the IEAD 100, is the manner in which an electrical connection is established between the ring electrode 120 and electronic circuitry carried inside of the IEAD 100. This electrical connection is established using a radial feed-through pin that fits within a recess formed in a segment of the edge of the case 124, as explained more fully below in connection with the description of FIGS. 5, 5A, 5B and 7.

In contrast to the feed-through pin that establishes electrical contact with the anode electrode, electrical connection with the cathode electrode 110 is established by simply forming or attaching the cathode electrode 110 to the front surface 106 of the IEAD case 124. In order to prevent the entire case 124 from functioning as the cathode (which is done to better control the electric fields established between the anode and cathode electrodes), the entire IEAD housing is covered in a layer of silicone molding 125 (see FIG. 7), except for the outside surface of the anode ring electrode 120 and the outside surface of the cathode electrode 110.

One of advantages of using a central cathode electrode and a ring anode electrode as described herein is that the electrode configuration is symmetrical. That is, when implanted, the surgeon or other medical personnel performing the implant surgery, need only assure that the cathode side of the IEAD 100, which (for the embodiment shown in FIGS. 1-7) is the front side 106 of the device, faces the target tissue location that is to receive the EA stimuli. The orientation of the IEAD 100 is otherwise not important.

Turning next to FIG. 17, a representation of a human patient is depicted showing the location of several acupoints on the patient's body. As the reference book WHO Standard Acupuncture Point Locations 2008 teaches, and as is known in the art, the acupoints shown in FIG. 17 represent only a very small number of the total number of acupoints that have been identified on the human body. Any of these acupoints (those shown and not shown in FIG. 17), as well as other target tissue locations, such as nerves or muscle fibers, could be designated as the target stimulation point for use by the IDEA described herein.

For example, FIG. 1A illustrates the location of an exemplary target tissue location 90 located on the skin surface of a limb 80 of the patient whereat the IEAD of FIG. 1 may be implanted for the treatment of a particular disease or condition. Such location is representative of a wide variety of target tissue locations where the IEAD of FIG. 1 could be implanted, some of which are located on limbs, others of which are located on other parts of the patient's body.

An implanted IEAD 100 is illustrated generally in FIGS. 1A and 1B. Shown in FIG. 1B is a sectional view of the limb 80, or other body tissue, wherein a target tissue location 90 has been identified that is to receive EA treatment using the IEAD 100. FIG. 1B. The target tissue location 90, e.g., an acupoint, is identified as being on the skin surface. An incision 84 (not shown in FIG. 1B, but which is shown in FIG. 1A) is made in the tissue 80 a short distance, e.g., 10-15 mm, away from the target location 90. A slot is formed at the incision 84 by lifting the skin closest to the acupoint up at the incision. As necessary, the surgeon may form a pocket under the skin at the acupoint location. The IEAD 100, with its top side 102 being closest to the skin (and thus also referred to as the "Skin Side"), is then slid through the slot 84 into the pocket so that the center of the IEAD is located under the target tissue location 90 on the skin surface. This implantation process may be as easy as inserting a coin into a slot. With the IEAD 100 in place, the incision is sewn or otherwise closed, leaving the IEAD 100 under the skin 80 at the target tissue location 90 where electroacupuncture (EA) stimulation is desired.

In this regard, it should be noted that while the target stimulation point is generally identified by an "acupoint," which is typically shown in drawings and diagrams as residing on the surface of the skin, the surface of the skin is not the actual target stimulation point. Rather, whether such stimulation comprises manual manipulation of a needle inserted through the skin at the location on the skin surface identified as an "acupoint", or whether such stimulation comprises electrical stimulation applied through an electrical field oriented to cause stimulation current to flow through the tissue at a prescribed depth below the acupoint location on the skin surface, the actual target tissue point to be stimulated is located beneath the skin at a depth d2 that varies depending on the particular acupoint location. When stimulation is applied at the target tissue point, such stimulation is effective at treating a condition or disease of the patient because there is something in the tissue at that target tissue location, or near that location, such as a nerve, a tendon, a muscle, or other type of tissue, that responds to the applied stimulation in a manner that contributes favorably to the treatment of the condition or disease experienced by the patient.

FIG. 1B illustrates a sectional view of the IEAD 100 implanted so as to be centrally located under the skin at the selected target tissue location 90, e.g., an acupoint 90, and over the acupoint axis line 92. The acupoint axis line 92 is a line that extends inwardly from the surface of the skin 80 in a direction that is more or less orthogonal to the skin surface at the location of the point 90 on the skin surface 80. Usually, for most patients, the IEAD 100 is implanted at a depth d1 of approximately 2-4 mm under the skin. The top (skin) side102 of the IEAD is nearest to the skin 80 of the patient. The bottom (cathode) side 106 of the IEAD, which is the side on which the central cathode electrode 110 resides, is farthest from the skin. Because the cathode electrode 110 is centered on the bottom of the IEAD, and because the IEAD 100 is implanted so as to be approximately centered under the location on the skin where the target acupoint 90 is located, the cathode 110 is also centered over the acupoint axis line 92.

FIG. 1B further illustrates the electric field gradient lines 88 that are created in the body tissue surrounding the acupoint 90 and the acupoint axis line 92. (Note: for purposes herein, when reference is made to providing EA stimulation at a specified acupoint, it is understood that the EA stimulation is provided at a depth of approximately d2 below the location on the skin surface where the acupoint is indicated as being located.) As seen in FIG. 1B, the electric field gradient lines are strongest along a line that coincides with, or is near to, the acupoint axis line 92. It is thus seen that one of the main advantages of using a symmetrical electrode configuration that includes a centrally located electrode 110 surrounded by an annular electrode 120 is that the precise orientation of the IEAD within its implant location is not important. So long as one electrode is centered over the desired target location, and the other electrode surrounds the first electrode (e.g., as an annular electrode), a strong electric field gradient is created that is aligned with the acupoint axis line. This causes the EA stimulation current to flow along (or very near) the acupoint axis line 92, and will result in the desired EA stimulation in the tissue at a depth d2 below the target location 90.

As can be seen from FIG. 17, some acupoints may be located on the head of the patient, e.g., acupoints GV20, or in other tissue locations where the patient's skull bone, or other skeletal structure, prevent implanting the IEAD 100 very deep below the skin. This situation is illustrated schematically in FIGS. 17A and 17B. As seen in these figures, a bone 89, e.g., the skull bone, is right under the skin 80, with not much tissue separating the two. These two figures assume that the actual desired target stimulation point is a nerve 87 (or some other tissue formation) between the underneath side of the skin 80 and the top surface of the bone 89. Hence, the challenge is to implant the IEAD 100 in a manner that provides effective EA stimulation at the desired target stimulation site, e.g., at the nerve 87 (or other tissue formation) that resides beneath the acupoint 90. FIGS 17A and 17B illustrate alternative methods for achieving this goal.

Shown in FIG. 17A is one alternative for implanting the IEAD 100 at an acupoint 90 located on the surface of the skin 80 above the bone 89, where the actual target stimulation point is a nerve 87, or some other tissue formation, that is located between the bone 89 and the underneath side of the skin 80. As shown in FIG. 17A, the IEAD 100 is implanted right under the skin with its front surface 106 facing down towards the target tissue location 87. This allows the electric fields (illustrated by the electric field gradient lines 88) generated by the IEAD 100 when EA stimulation pulses are to be generated to be most heavily concentrated at the target tissue stimulation site 87. These electric field gradient lines 88 are established between the two electrodes 110 and 120 of the IEAD. For the embodiment shown here, these two electrodes comprise a ring electrode 120, positioned around the perimeter edge of the IEAD housing, and a central electrode 110, positioned in the center of the front surface 102 of the IEAD housing. These gradient lines 88 are most concentrated right below the central electrode, which is where the target tissue location 87 resides. Hence, the magnitude of the electrical stimulation current will also be most concentrated at the target tissue location 87, which is the desired result.

FIG. 17B shows another alternative for implanting the IEAD 100 at the acupoint 90 located on the surface of the skin 80 above the bone 89, where the actual target stimulation point is a nerve 87, or some other tissue formation, that is located between the bone 89 and the underneath side of the skin 80. As shown in FIG. 17B, the IEAD 100 is implanted in a pocket 81 formed in the bone 89 at a location underneath the acupoint 90. In this instance, and as the elements are oriented in FIG. 17B, the front surface 106 of the IEAD 100 faces upwards towards the target tissue location 87. As with the implant configuration shown in FIG. 17A, this configuration also allows the electric fields (illustrated by the electric field gradient lines 88) that are generated by the IEAD 100 when EA stimulation pulses are generated to be most heavily concentrated at the target tissue stimulation site 87.

There are advantages and disadvantages associated with each of the two alternative implantation configurations shown in FIGS. 17A and 17B. Generally, the implantation procedure used to achieve the configuration shown in FIG. 17A is a simpler procedure with less risk. That is, all that need to be done by the surgeon to implant that EA device 100 as shown in FIG. 17A is to make an incision 82 in the skin 80 a short distance, e.g., 10-15 mm, away from the acupoint 90. This incision should be made parallel to the nerve 87 so as to minimize the risk of cutting the nerve 87. A slot is then formed at the incision by lifting the skin closest to the acupoint up at the incision and by carefully sliding the IEAD 100, with its front side 102 facing the skull, into the slot so that the center of the IEAD is located under the acupoint 90. Care is taken to assure that the nerve 87 resides below the front surface of the IEAD 100 as the IEAD is slid into position.

In contrast, if the implant configuration shown in FIG. 17B is to be used, then the implant procedure is somewhat more complicated with somewhat more risk. That is, to achieve the implant configuration shown in FIG. 17B, a sufficiently large incision must be made in the skin at the acupoint 90 to enable the skin 80 to be peeled or lifted away to expose the surface of the bone so that the cavity 81 may be formed in the bone. While doing this, care must be exercised to hold the nerve 87 (or other sensitive tissue areas) away from the cutting tools used to form the cavity 81. Once the cavity 81 is formed, the IEAD 100 is laid in the cavity, with its front surface 102 facing upward, the nerve 87 (and other sensitive tissue areas) are carefully repositioned above the IEAD 100, and the skin is sewn or clamped to allow the incision to heal. In this unique situation, where a cavity is formed in the bone to hold the IEAD 100, the back side 102 of the IEAD 100 (which sometimes is called the "skin" side), is actually farthest away from the skin surface.

However, while the surgical procedure and attendant risks may be more complicated when the configuration of FIG. 17B is employed, the final results of the configuration of FIG. 17B may be more aesthetically pleasing to the patient than are achieved with the configuration of FIG. 17A. That is, given the shallow space between the skin and the bone at an acupoint above the bone, the implant configuration of FIG. 17A will likely result in a small hump or bump at the implant site.

Insofar as Applicant is aware at the present time, of the two implant configurations shown in FIGS. 17A and 17B, there is no theoretical performance advantage that one implant configuration provides over the other. That is, both implant configurations should perform equally well insofar as providing EA stimulation pulses at the desired target tissue location 87 is concerned.

Thus, which implant configuration is used will, in large part, be dictated by individual differences in patient anatomy, patient preference, and surgeon preferences and skill levels.

From the above, it is seen that one of the main advantages of using a symmetrical electrode configuration that includes a centrally located electrode surrounded by an annular electrode, as is used in the embodiment described in connection with FIGS. 1-7, is that the precise orientation of the IEAD 100 within its implant location is not important. So long as one electrode faces and is centered over the desired target location, and the other electrode surrounds the first electrode (e.g., as an annular electrode), a strong electric field gradient is created that is aligned with the desired target tissue location. This causes the EA stimulation current to flow at (or very near to) the target tissue location 87.

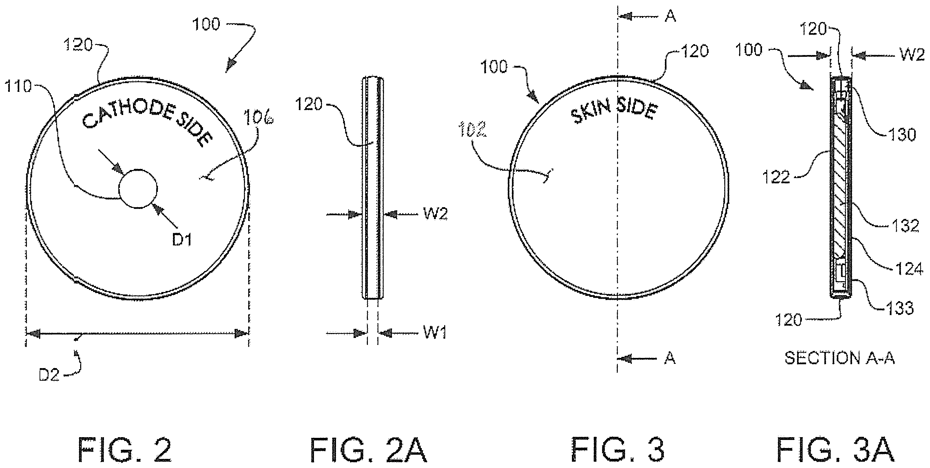

FIG. 2 shows a plan view of the "front" (or "cathode") side 106 of the IEAD 100. As seen in FIG. 2, the cathode electrode 110 appears as a circular electrode, centered on the front side, having a diameter D1. The IEAD housing has a diameter D2 and an overall thickness or width W2. For the preferred embodiment shown in these figures, D1 is about 4 mm, D2 is about 23 mm and W2 is a little over 2 mm (2.2 mm).

FIG. 2A shows a side view of the IEAD 100. The ring anode electrode 120, best seen in FIG. 2A, has a width W1 of about 1.0 mm, or approximately ½ of the width W2 of the IEAD.

FIG. 3 shows a plan view of the "back" (or "skin") side 102 of the IEAD 100. As will be evident from subsequent figure descriptions, e.g., FIGS. 5A and 5B, the back side 102 of the IEAD 100 comprises a cover plate 122 that is welded in place once the bottom case 124 has all of the electronic circuitry, and other components, placed inside of the housing.

FIG. 3A is a sectional view of the IEAD 100 of FIG. 1 taken along the line A-A of FIG. 3. Visible in this sectional view is the feed-through pin 130, including the distal end of the feed-through pin 130 attached to the ring anode electrode 120. Also visible in this section view is an electronic assembly 133 on which various electronic components are mounted, including a disc-shaped coin-cell battery 132. FIG. 3A further illustrates how the cover plate 122 is welded, or otherwise bonded, to the bottom case 124 in order to form the hermetically-sealed IEAD housing 100.

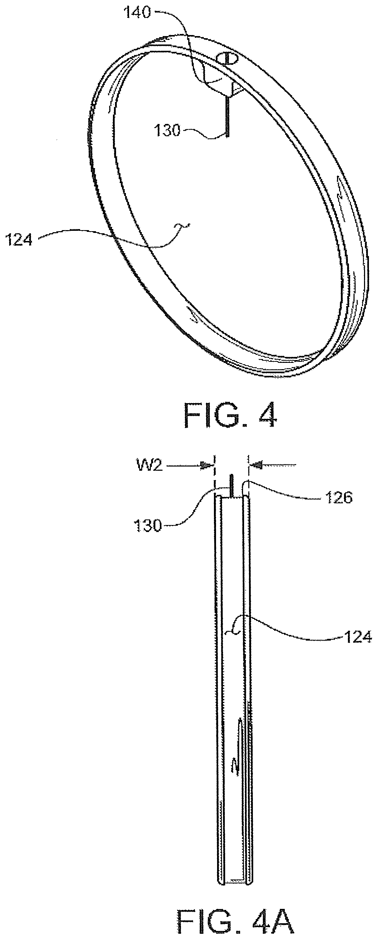

FIG. 4 shows a perspective view of the IEAD case 124, including the feed-through pin 130, before the electronic components are placed therein, and before being sealed with the "skin side" cover plate 122. The case 124 is similar to a shallow "can" without a lid, having a short side wall around its perimeter. Alternatively, the case 124 may be viewed as a short cylinder, closed at one end but open at the other. (Note, in the medical device industry the housing of an implanted device is often referred to as a "can".) The feed-through pin 130 passes through a segment of the wall of the case 124 that is at the bottom of a recess 140 formed in the wall. The use of this recess 140 to hold the feed-through pin 130 is a key feature of the invention because it keeps the temperature-sensitive portions of the feed-through assembly (those portions that could be damaged by excessive heat or mechanical shock) away from the thermal shock and residual weld stress inflicted upon the case 124 when the cover plate 122 is welded thereto.

While the case 124 may be viewed as a "short cylinder," as indicated above, it is important to note that the overall shape and size of the case is more properly described as disc, or coin-shaped, having a diameter D2 that is several times greater, e.g., 10 times greater, than its thickness W2. Thus, the ratio of W2 to D2 is usually on the order of 0.12 or less. This thin, disc shape, along with its leadless symmetrical electrode configuration, advantageously allows the EA device housing, including its electrodes, to be readily slid into a desired tissue implant location at the desired target tissue location in a very noninvasive manner.

This overall disc-shaped configuration, with a ratio of W2/D2 less than or equal to 0.12, further distinguishes the invention from the long cylindrical-shaped microstimulator devices, such as those described in the Whitehurst patents referenced above in Paragraphs **[0021]** and **[0022].** These cylindrical-shaped devices not only have a ratio of W2/D2 much greater than 1.0, but they also do not employ a symmetrical electrode configuration of the type disclosed herein, nor do they use a thin, high impedance primary coin-cell battery as disclosed herein. Thus, these cylindrical-shaped devices fall far short of offering the same advantages as are offered by the EA device described herein by virtue of its thin disc-shape, symmetrical electrode configuration and use of a high impedance coin cell primary battery.

FIG. 4A is a side view of the IEAD case 124, and shows an annular rim 126 formed on both sides of the case 124. The ring anode electrode 120 fits between these rims 126 once the ring electrode 120 is positioned around the edge of the case 124. (This ring electrode 120 is, for most configurations, used as an anode electrode. Hence, the ring electrode 120 may sometimes be referred to herein as a ring anode electrode. However, it is noted that the ring electrode could also be employed as a cathode electrode, if desired.) A silicone insulator layer 129 (see Fig. 7) is placed between the backside of the ring anode electrode 120 and the perimeter edge of the case 124 where the ring anode electrode 120 is placed around the edge of the case 124.

FIG. 5 shows a plan view of the empty IEAD case 124 shown in the perspective view of FIG. 4. An outline of the recess cavity 140 is also seen in FIG. 5, as is the feed-through pin 130. A bottom edge of the recess cavity 140 is located a distance D5 radially inward from the edge of the case 124. In one embodiment, the distance D5 is between about 2.0 to 2.5 mm. The feed-through pin 130, which is just a piece of solid wire, is shown in FIG. 5 extending radially outward from the case 124 above the recess cavity 140 and radially inward from the recess cavity towards the center of the case 124. The length of this feed-through pin 130 is trimmed, as needed, when a distal end (extending above the recess) is connected (welded) to the anode ring electrode 120 (passing through a hole in the ring electrode 120 prior to welding) and when a proximal end of the feed-through pin 130 is connected to an output terminal of the electronic assembly 133.

FIG. 5A depicts a sectional view of the IEAD housing 124 of FIG. 5 taken along the section line A-A of FIG. 5. FIG. 5B shows an enlarged view or detail of the portion of FIG. 5A that is encircled with the line B. Referring to FIGS. 5A and 5B jointly, it is seen that the feed-through pin 130 is embedded within an insulator material 136, which insulating material 136 has a diameter of D3. The feed-through pin assembly (which pin assembly comprises the combination of the pin 130 embedded into the insulator material 136) resides on a shoulder around an opening or hole formed in the bottom of the recess 140 having a diameter D4. For the embodiment shown in FIG. 5A and 5B, the diameter D3 is 0.95 - 0.07 mm, where the -0.07 mm is a tolerance. (Thus, with the tolerance considered, the diameter D3 may range from 0.88 mm to 0.95 mm) The diameter D4 is 0.80 mm with a tolerance of - 0.06 mm. (Thus, with the tolerance considered, the diameter D4 could range from 0.74 mm to 0.80 mm).

The feed-through pin 130 is preferably made of pure platinum 99.95%. A preferred material for the insulator material 136 is Ruby or alumina. The IEAD case124, and the cover 122, are preferably made from titanium. The feed-through assembly, including the feed-through pin 130, ruby/alumina insulator 136 and the case 124 are hermetically sealed as a unit by gold brazing. Alternatively, active metal brazing can be used. (Active metal brazing is a form of brazing which allows metal to be joined to ceramic without metallization.)

The hermeticity of the sealed IEAD housing is tested using a helium leak test, as is common in the medical device industry. The helium leak rate should not exceed 1x10⁻⁹ STD cc/sec at 1 atm pressure. Other tests are performed to verify the case-to-pin resistance (which should be at least 15x10⁶ Ohms at 100 volts DC), the avoidance of dielectric breakdown or flashover between the pin and the case 124 at 400 volts AC RMS at 60 Hz and thermal shock.

One important advantage provided by the feed-through assembly shown in FIGS. 4A, 5, 5A and 5B is that the feed-through assembly made from the feed-through pin 130, the ruby insulator 136 and the recess cavity 140 (formed in the case material 124) may be fabricated and assembled before any other components of the IEAD 100 are placed inside of the IEAD case 124. This advantage greatly facilitates the manufacture of the IEAD device.

Next, with reference to FIGS. 5C and 5D, some alternate embodiments showing the use of multiple feed-through pins with the case 124 are illustrated. Two such embodiments are shown. In a first alternate embodiment, shown in FIG. 5C, multiple recess cavities 140 are formed in the case 124. That is, as seen in FIG. 5C, and with respect to the orientation of the case shown in FIG. 5C, a first recess cavity 140a is on the left side of the case (approximately at the 11:00 o'clock position), a second recess cavity 140b is in the middle of the case (approximately at the 12:00 o'clock position), and a third recess cavity 140c is on the right side of the case (approximately at the 1:00 o'clock position). Each of these multiple recess cavities, 140a, 140b and 140c, have a respective feed-through pin 130, with insulator material 136, passing through an opening in the bottom of their respective recess cavities. When initially assembled, each feed-through pin 130 has a distal tip 131 that extends radially outward beyond the perimeter edge of the case 124. The distal tip 131 can be trimmed to a suitable length, as needed, in order to connect it to its electrode. Likewise, a proximal end 129' of each feed-through pin 130 extends radially inward toward the center of the case 124. This proximal end 129' can similarly be trimmed, as required, in order to connect it to an appropriate location on the electronic assembly 133 or other location within the case 124.

In a second alternate embodiment, shown in FIG. 5D, multiple feed-through pins 130 are placed through a respective opening in the bottom of a single recess cavity 141. Two such feed-through pins 130, each with respective insulator material 136, are illustrated in FIG. 5D, but this number is only exemplary.

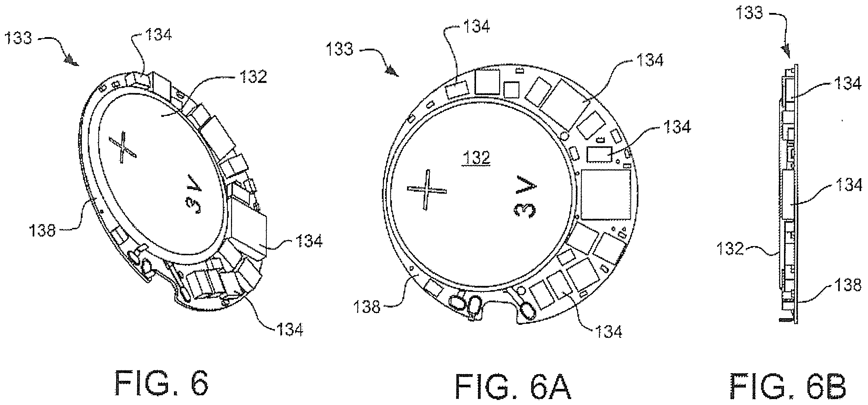

Turning next to FIG. 6, there is shown a perspective view of an electronic assembly 133. The electronic assembly 133 includes a multi-layer printed circuit (PC) board 138, or equivalent mounting structure, on which a battery 132 and various electronic components 134 are mounted. The battery 132 comprises a thin, coin-cell battery having a diameter that is no greater than about ¾ D2. Where D2 is 25 mm, the diameter of the coin cell battery is thus no greater than about 18 mm. This assembly is adapted to fit inside of the empty bottom housing 124 of FIG. 4 and FIG. 5.

FIGS. 6A and 6B show a plan view and side view, respectively, of the electronic assembly 133 shown in FIG. 6. The electronic components are assembled and connected together so as to perform the circuit functions needed for the IEAD 100 to perform its intended functions. These circuit functions are explained in more detail below under the sub-heading "Electrical Design".

FIG. 7 shows an exploded view of the complete IEAD 100, illustrating its main constituent parts. As seen in FIG. 7, the IEAD 100 includes, starting on the right and going left, a cathode electrode 110, a ring anode electrode 120, an insulating layer 129, the bottom case 124 (the "can" portion of the IEAD housing, and which includes the feed-through pin 130 which passes through an opening in the bottom of the recess 140 formed as part of the case, but wherein the feed-through pin 130 is insulated and does not make electrical contact with the metal case 124 by the ruby insulator 136), the electronic assembly 133 (which includes the battery 132 and various electronic components 134 mounted on a pc board 138) and the cover plate 122. The cover plate 122 is welded to the edge of the bottom case 124 using laser beam welding, or some equivalent process, as one of the final steps in the assembly process.

Other components included in the IEAD assembly, but not necessarily shown or identified in FIG. 7, include adhesive patches for bonding the battery 132 to the PC board 138 of the electronic assembly 133, and for bonding the electronic assembly 133 to the inside of the bottom of the case 124. To prevent high temperature exposure of the battery 132 during the assembly process, conductive epoxy is used to connect a battery terminal to the PC board 138. Because the curing temperature of conductive epoxy is 125 °C, the following process is used: (a) first cure the conductive epoxy of a battery terminal ribbon to the pc board without the battery, (b) then glue the battery to the pc board using room temperature cure silicone, and (c) laser tack weld the connecting ribbon to the battery.

Also not shown in FIG. 7 is the manner of connecting the proximal end of the feed-through pin 130 to the PC board 138, and connecting a PC board ground pad to the case 124. A preferred method of making these connections is to use conductive epoxy and conductive ribbons, although other connection methods known in the art may also be used.

Further shown in FIG. 7 is a layer of silicon molding 125 that is used to cover all surfaces of the entire IEAD 100 except for the anode ring electrode 120 and the circular cathode electrode 110. An overmolding process is used to accomplish this, although overmolding using silicone LSR 70 (curing temperature of 120 °C) with an injection molding process cannot be used. Overmolding processes that may be used include: (a) molding a silicone jacket and gluing the jacket onto the case using room temperature cure silicone (RTV) inside of a mold, and curing at room temperature; (b) injecting room temperature cure silicone in a PEEK or Teflon® mold (silicone will not stick to the Teflon® or PEEK material); or (c) dip coating the IEAD 100 in room temperature cure silicone while masking the electrode surfaces that are not to be coated. (Note: PEEK is a well-known semicrystalline thermoplastic with excellent mechanical and chemical resistance properties that are retained at high temperatures.)

When assembled, the insulating layer 129 is positioned underneath the ring anode electrode 120 so that the anode electrode does not short to the case 124. The only electrical connection made to the anode electrode 120 is through the distal tip of the feed-through pin 130. The electrical contact with the cathode electrode 110 is made through the case 124. However, because the entire IEAD is coated with a layer of silicone molding 125, except for the anode ring electrode 120 and the circular cathode electrode 110, all stimulation current generated by the IEAD 100 must flow between the exposed surfaces of the anode and cathode.

It is noted that while the preferred configuration described herein uses a ring anode electrode 120 placed around the edges of the IEAD housing, and a circular cathode electrode 110 placed in the center of the cathode side of the IEAD case 124, such an arrangement could be reversed, i.e., the ring electrode could be the cathode, and the circular electrode could be the anode.

Moreover, the location and shape of the electrodes may be configured differently than is shown in the one preferred embodiment described above in connection with FIGS. 1, and 2-7. For example, the ring anode electrode 120 need not be placed around the perimeter of the device, but such electrode may be a flat circumferential electrode that assumes different shapes (e.g., round or oval) that is placed on the front or back surface of the IEAD so as to surround the central electrode. Further, for some embodiments, the surfaces of the anode and cathode electrodes may have convex surfaces.

The invention incorporates a round, or short cylindrical-shaped housing, also referred to as a coin-shaped or disc-shaped housing These shapes permit the basic principles of the invention to be used to provide a robust, compact, thin, case to house the electronic circuitry and power source used by the invention; as well as to help protect a feed-through assembly from being exposed to excessive heat during assembly, and to allow the thin device to provide the benefits described herein related to its manufacture, implantation and use. For example, as long as the device remains relatively thin, e.g., with a thickness L2 no more than about 2-3 mm, and does not have a maximum linear dimension L1, where L1 and L2 are measured in planes that are generally orthogonal to each other, greater than about 25 mm, so that the ratio of L2/L1 is on the order of 0.12 or less, then the device can be readily implanted in a pocket over the tissue area where the selected target tissue location(s) is located. As long as there is a recess in the wall around the perimeter of the case wherein the feed-through assembly may be mounted, which recess effectively moves the wall or edge of the case inwardly into the housing a safe thermal distance, as well as a safe residual weld stress distance, from the perimeter wall where a hermetically-sealed weld occurs, the principles of the invention apply.

Further, it should be noted that while the preferred configuration of the IEAD described herein utilizes a central electrode on one of its surfaces that is round, having a diameter of nominally 4 mm, such central electrode need not necessarily be round. It could be oval shaped, polygonal-shaped, or shaped otherwise, in which case its size is best defined by its maximum width, which will generally be no greater than about 7 mm.

Finally, it is noted that the electrode arrangement may be modified somewhat, and the desired attributes of the invention may still be achieved. For example, as indicated previously, one preferred electrode configuration for use with the invention utilizes a symmetrical electrode configuration, e.g., an annular electrode of a first polarity that surrounds a central electrode of a second polarity. Such a symmetrical electrode configuration makes the implantable EA device relatively immune to being implanted in an improper orientation relative to the body tissue at the selected target tissue location(s), e.g., acupoint(s), that is/are being stimulated. However, an electrode configuration that is not symmetrical may still be used and at least some of the therapeutic effects of the invention may still be achieved. For example, two spaced-apart electrodes on a front surface of the housing, one of a first polarity, and a second of a second polarity, could still, when oriented properly with respect to a selected target tissue location, provide some desired therapeutic results.

FIG. 7A schematically illustrates a few alternative electrode configurations that may be used with the invention. The electrode configuration schematically shown in the upper left corner of FIG. 7A, identified as "I", schematically illustrates one central electrode 110 surrounded by a single round ring electrode 120. This is one of the preferred electrode configurations that has been described previously in connection, e.g., with the description of FIGS. 1, 1A, 1B and 7, and is presented in FIG. 7A for reference and comparative purposes.

In the lower left corner of FIG. 7A, identified as "II", an electrode/array configuration is schematically illustrated that has a central electrode 310 of a first polarity surrounded by an oval-shaped electrode array 320a of two electrodes of a second polarity. (This oval-shaped array 320a could also be round.) When the two electrodes (of the same polarity) in the electrode array 320a are properly aligned with the body tissue being stimulated, e.g., aligned with a nerve underlying the desired target tissue location, e.g., acupoint, then such electrode configuration can stimulate the body tissue (e.g., the underlying nerve) at or near the desired tissue target(s) with the same, or almost the same, efficacy as can the electrode configuration I (upper right corner of FIG. 7A).

Note, as has already been described above, the phrase "electrode or electrode array," or "electrodes or electrode arrays," may also be referred to herein as "electrode/array" or "electrodes/arrays," respectively. For the ease of explanation, when an electrode array is referred to herein that comprises a plurality (two or more) of individual electrodes of the same polarity, the individual electrodes of the same polarity within the electrode array may also be referred to as "individual electrodes", "segments" of the electrode array, "electrode segments", or just "segments".

In the lower right corner of FIG. 7A, identified as "III", en electrode configuration is schematically illustrated that has a round central electrode/array 310b of three electrode segments of a first polarity surrounded by an oval electrode array 320b of three electrode segments of a second polarity. (These round or oval shapes could be altered, as desired or needed. That is, the round central electrode/array 310b could be an oval-shaped electrode array, and the oval-shaped electrode array 320b could be a round electrode array.) As shown in configuration III of FIG. 7A, the three electrode segments of the electrode array 320b are symmetrically positioned within the array 320b, meaning that they are positioned more or less equidistant from each other. However, a symmetrical positioning of the electrode segments within the array is not always necessary to stimulate the body tissue at the desired target tissue location(s) with some efficacy.

In the upper right corner of FIG. 7A, identified as "IV", an electrode/array configuration is schematically illustrated that has a central electrode array 310c of a first polarity surrounded by an oval electrode array 320c of four electrode segments of a second polarity. The four electrode segments of the electrode array 320c are arranged symmetrically in a round or oval-shaped array. The four electrode segments of the electrode array 310c are likewise arranged symmetrically in a round or oval-shaped array. While preferred for many configurations, the use of a symmetrical electrode/array, whether as a central electrode array 310 or as a surrounding electrode/array 320, is not always required.

The electrode configurations I, II, III and IV shown schematically in FIG. 7A are only representative of a few electrode configurations that may be used with the present invention. Further, it is to be noted that the central electrode/array 310, 310b or 310c need not have the same number of electrode segments as does the surrounding electrode/array 320a, 320b or 320c. Typically, the central electrode/array 310, 310b or 310c of a first polarity will be a single electrode; whereas the surrounding electrode/array 320a, 320b or 320c of a second polarity may have *n* individual electrode segments, where *n* is an integer that can vary from 1, 2, 3, *...n.* Thus, for a circumferential electrode array where *n* = 4, there are four electrode segments of the same polarity arranged in circumferential pattern around a central electrode/array. If the circumferential electrode array with *n* = 4 is a symmetrical electrode array, then the four electrode segments will be spaced apart equally in a circumferential pattern around a central electrode/array. When *n* = 1, the circumferential electrode array reduces to a single circumferential segment or a single annular electrode that surrounds a central electrode/array.

Additionally, the polarities of the electrode/arrays may be selected as needed. That is, while the central electrode/array 310, 310b or 310c is typically a cathode (-), and the surrounding electrode/array 320a, 320b or 320c is typically an anode (+), these polarities may be reversed.

As has already been mentioned, the shape of the circumferential electrode/array, whether circular, oval, or other shape, need not necessarily be the same shape as the IEAD housing, unless the circumferential electrode/array is attached to a perimeter edge of the IEAD housing. The IEAD housing may be round, or it may be oval, or it may have a polygon shape, or other shape, as needed to suit the needs of a particular manufacturer and/or patient.

Next, with reference to FIGS. 8-16, the electrical design and operation of the circuits employed within the IEAD 100 will be described. These circuits allow a thin, high impedance, coin-cell type battery to be employed within the IEAD to provide its operating power over a long period of time.

FIG. 8 shows a functional block diagram of an implantable electroacupuncture device (IEAD) 100 made in accordance with the teachings disclosed herein. As seen in FIG. 8, the IEAD 100 uses an implantable primary battery 215 having a battery voltage V_{BAT}. In one preferred embodiment, this primary battery 215 comprises a lithium battery having a nominal output voltage of 3 V, such as the CR1612 battery manufactured by Panasonic. Also included within the IEAD 100 is a Boost Converter circuit 200, an Output Circuit 202 and a Control Circuit 210. The primary battery 115, boost converter circuit 200, output circuit 202 and control circuit 210 are all housed within an hermetically sealed housing 124.

Note, that a "primary" battery, for purposes of this application, means a non-rechargeable battery. The chemistry and structure used within rechargeable batteries is different from that used within non-rechargeable batteries. A non-rechargeable battery can be made smaller, especially thinner, than can a rechargeable battery having approximately the same nominal output voltage. Also, additional circuitry is needed inside of an implantable device that uses a rechargeable battery, which takes up additional space, not to mention the external recharging circuitry that must be used to couple recharging power into the rechargeable battery within the implanted device. Because one key feature of the IEAD device disclosed herein is to be packaged within a small, especially thin, coin-shaped housing, and because other key features include keeping the IEAD circuits simple and inexpensive, a primary battery (not a rechargeable battery) is thus used.

As controlled by the control circuit 210, the output circuit 202 of the IEAD 100 generates a sequence of stimulation pulses that are delivered to electrodes E1 and E2, through feed-through terminals 206 and 207, respectively, in accordance with a prescribed stimulation regimen. A coupling capacitor C_{C} is also employed in series with at least one of the feed-through terminals 206 or 207 to prevent DC (direct current) current from flowing into the patient's body tissue.

As explained more fully below in connection with the description of FIGS. 15A and 15B, the prescribed stimulation regimen typically comprises a continuous stream of stimulation pulses having a fixed amplitude, e.g., V_{A} volts, a fixed pulse width, e.g., 0.5 millisecond, and at a fixed frequency, e.g., 2 Hz, during each stimulation session. The stimulation session, also as part of the stimulation regimen, is generated at a very low duty cycle, e.g., for 30 minutes once each week. Other stimulation regimens may also be used, e.g., using a variable frequency for the stimulus pulse during a stimulation session rather than a fixed frequency. Also, the rate of occurrence of the stimulation session may be varied from as short as, e.g., 1 day, to as long as, e.g., 14 days.

In one preferred embodiment, the electrodes E1 and E2 form an integral part of the housing 124. That is, electrode E2 may comprise a circumferential anode electrode that surrounds a cathode electrode E1. The cathode electrode E1, for the embodiment described here, is electrically connected to the case 124 (thereby making the feed-through terminal 206 unnecessary). Using this electrode arrangement, the stimulation pulses delivered to the target tissue location through the electrodes E1 and E2 are, relative to a zero volt ground (GND) reference, negative stimulation pulses, as shown in the waveform diagram near the lower right hand corner of FIG. 8A.

Thus, in the embodiment described in FIG. 8, it is seen that during a stimulation pulse the electrode E2 functions as an anode, or positive (+) electrode, and the electrode E1 functions as a cathode, or negative (-) electrode.

The battery 115 provides all of the operating power needed by the EA device 100. The battery voltage V_{BAT} is not the optimum voltage needed by the circuits of the EA device, including the output circuitry, in order to efficiently generate stimulation pulses of sufficient amplitude, e.g., -V_{A} volts. The amplitude V_{A} of the stimulation pulses is typically many times greater than the battery voltage V_{BAT}. This means that the battery voltage must be "boosted", or increased, in order for stimulation pulses of amplitude V_{A} to be generated. Such "boosting" is done using the boost converter circuit 200. That is, it is the function of the Boost Converter circuit 200 to take its input voltage, V_{BAT}, and convert it to another voltage, e.g., V_{OUT}, which voltage V_{OUT} is needed by the output circuit 202 in order for the IEAD 100 to perform its intended function.

The IEAD 100 shown in FIG. 8, and packaged as described above in connection with FIGS. 1-7A, advantageously provides a tiny self-contained, coin-sized stimulator that may be implanted in a patient at or near a specified acupoint in order to favorably treat a condition or disease of a patient. The coin-sized stimulator advantageously generates and applies electrical stimulation pulses at very low levels and low duty cycles in accordance with specified stimulation regimens through electrodes that form an integral part of the housing of the stimulator. A tiny coin-cell type battery inside of the coin-sized stimulator provides enough energy for the stimulator to carry out its specified stimulation regimen over a period of several years, despite the fact that the battery typically has a relatively high battery impedance, e.g., greater than 5 ohms, and often as high as 150 ohms, or more. Thus, the coin-sized stimulator, once implanted, provides an unobtrusive, needleless, long-lasting, safe, elegant and effective mechanism for treating certain conditions and diseases that have long been treated by acupuncture or electroacupuncture.

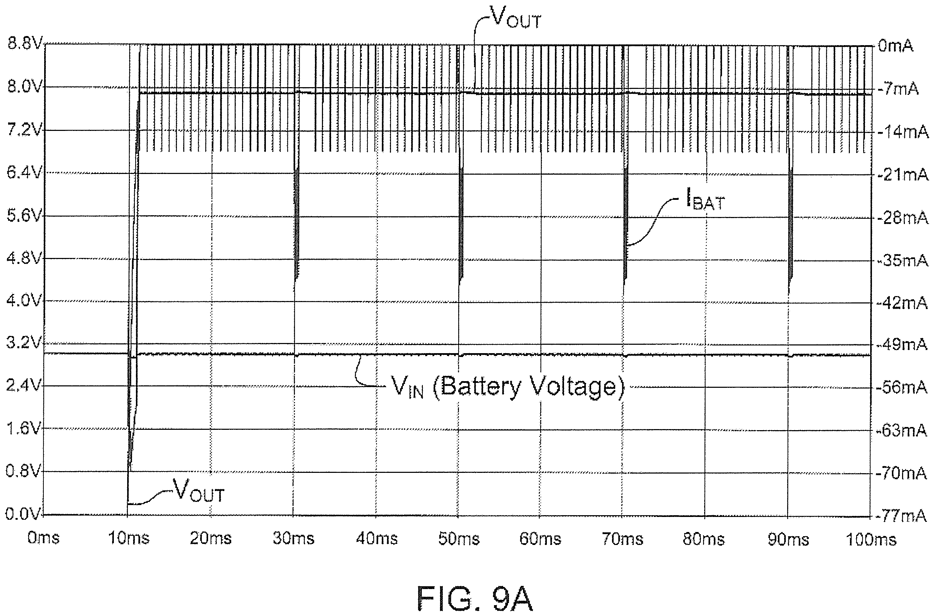

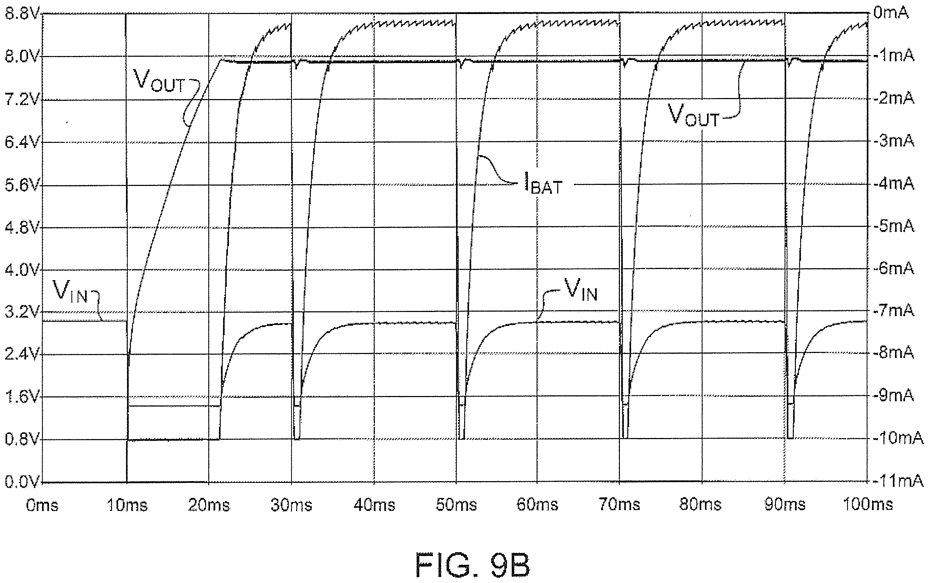

A boost converter integrated circuit (IC) typically draws current from its power source in a manner that is proportional to the difference between the actual output voltage V_{OUT} and a set point output voltage, or feedback signal. A representative boost converter circuit that operates in this manner is shown in FIG. 9. At boost converter start up, when the actual output voltage is low compared to the set point output voltage, the current drawn from the power source can be quite large. Unfortunately, when batteries are used as power sources, they have internal voltage losses (caused by the battery's internal impedance) that are proportional to the current drawn from them. This can result in under voltage conditions when there is a large current demand from the boost converter at start up or at high instantaneous output current. Current surges and the associated under voltage conditions can lead to undesired behavior and reduced operating life of an implanted electro-acupuncture device.

In the boost converter circuit example shown in FIG. 9, the battery is modeled as a voltage source with a simple series resistance. With reference to the circuit shown in FIG. 9, when the series resistance R_{BAT} is small (5 Ohms or less), the boost converter input voltage V_{IN}, output voltage V_{OUT} and current drawn from the battery, I_{BAT}, typically look like the waveform shown in FIG. 9A, where the horizontal axis is time, and the vertical axis on the left is voltage, and the vertical axis of the right is current.

Referring to the waveform in FIG. 9A, at boost converter startup (10 ms), there is 70 mA of current drawn from the battery with only ∼70 mV of drop in the input voltage V_{IN}. Similarly, the instantaneous output current demand for electro-acupuncture pulses draws up to 40 mA from the battery with an input voltage drop of -40 mV.

Disadvantageously, however, a battery with higher internal impedance (e.g., 160 Ohms), cannot source more than a milliampere or so of current without a significant drop in output voltage. This problem is depicted in the timing waveform diagram shown in FIG. 9B. In Fig. 9B, as in Fig. 9A, the horizontal axis is time, the left vertical axis is voltage, and the right vertical axis is current.

As seen in FIG. 9B, as a result of the higher internal battery impedance, the voltage at the battery terminal (V_{IN}) is pulled down from 2.9 V to the minimum input voltage of the boost converter (∼1.5 V) during startup and during the instantaneous output current load associated with electro-acupuncture stimulus pulses. The resulting drops in output voltage V_{OUT} are not acceptable in any type of circuit except an uncontrolled oscillator circuit.

Also, it should be noted that although the battery used in the boost converter circuit is modeled in FIG. 9 as a simple series resistor, battery impedance can arise from the internal design, battery electrode surface area and different types of electrochemical reactions. All of these contributors to battery impedance can cause the voltage of the battery at the battery terminals to decrease as the current drawn from the battery increases.

In a suitably small and thin implantable electroacupuncture device (IEAD) of the type disclosed herein, it is desired to use a higher impedance battery in order to assure a small and thin device, keep costs low, and/or to have low self-discharge rates. The battery internal impedance also typically increases as the battery discharges. This can limit the service life of the device even if a new battery has acceptably low internal impedance. Thus, it is seen that for the IEAD 100 disclosed herein to reliably perform its intended function over a long period of time, a circuit design is needed for the boost converter circuit that can manage the instantaneous current drawn from the battery. Such current management is needed to prevent the battery's internal impedance from causing V_{IN} to drop to unacceptably low levels as the boost converter circuit pumps up the output voltage, V_{OUT}, and when there is high instantaneous output current demand, as occurs when stimulation pulses are generated.

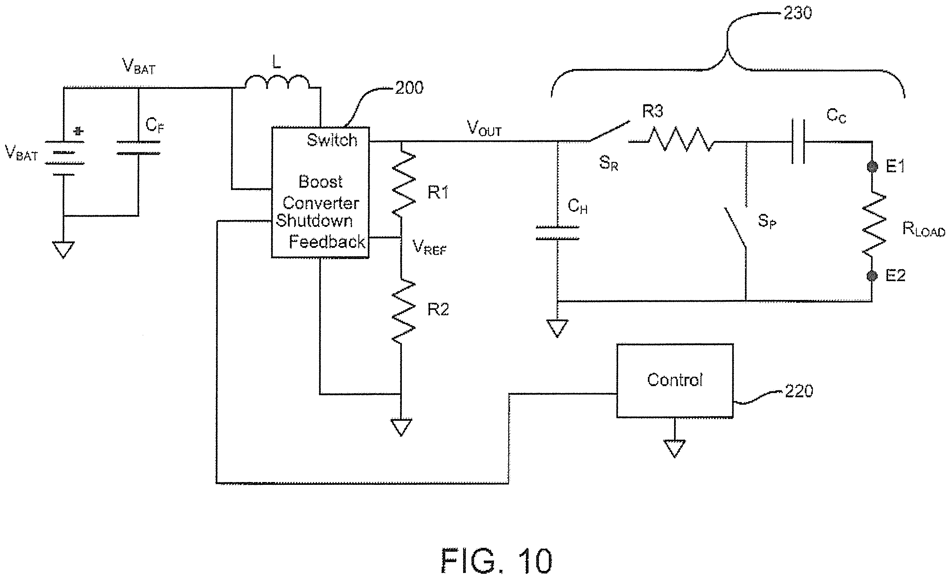

To provide this needed current management, the IEAD 100 disclosed herein employs electronic circuitry as shown in FIG. 10, or equivalents thereof. Similar to what is shown in FIG. 8, the circuitry of FIG. 10 includes a battery, a boost converter circuit 200, an output circuit 230, and a control circuit 220. The control circuit 220 generates a digital control signal that is used to duty cycle the boost converter circuit 200 ON and OFF in order to limit the instantaneous current drawn from the battery. That is, the digital control signal pulses the boost converter ON for a short time, but then shuts the boost converter down before a significant current can be drawn from the battery. In conjunction with such pulsing, an input capacitance C_{F} is used to reduce the ripple in the input voltage V_{IN}. The capacitor C_{F} supplies the high instantaneous current for the short time that the boost converter is ON and then recharges more slowly from the battery during the interval that the boost converter is OFF.

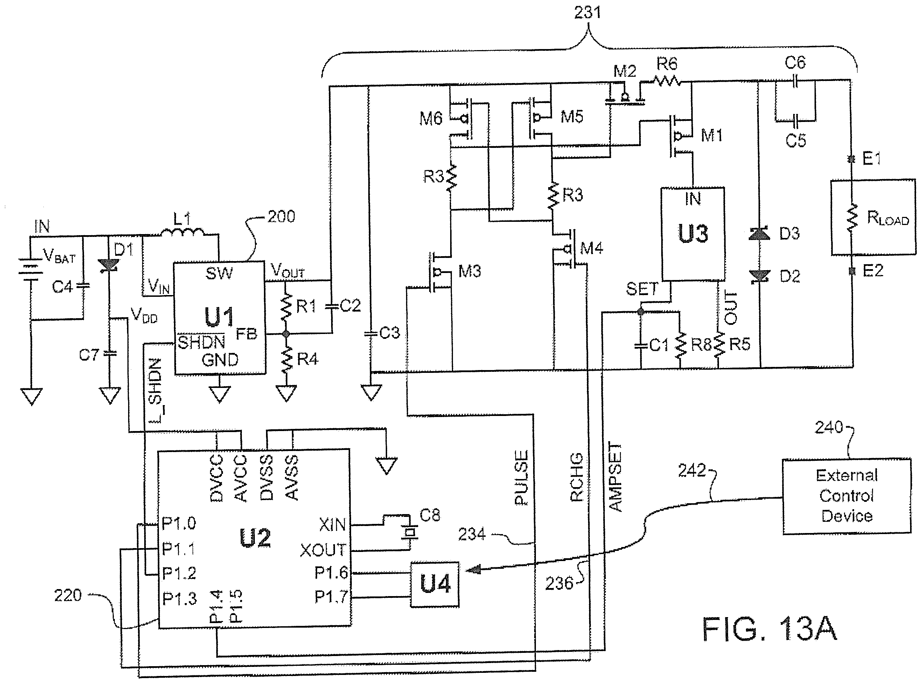

A variation of the above-described use of a digital control signal to duty cycle the boost converter circuit 200 ON and OFF is to let the digital control be generated within the boost converter 200 itself (without having to use a separate control circuit 220). In accordance with this variation, the boost converter circuit 200 shuts itself down whenever the battery voltage falls below a predetermined level above that required by the remaining circuitry. For example, the MAX8570 boost converter IC, commercially available from Maxim, shuts down when the applied voltage falls below 2.5 V. This is still a high enough voltage to ensure the microprocessor and other circuitry remain operational. Thus, as soon as the input voltage drops below 2.5 volts, the boost converter circuit shuts down, thereby limiting the instantaneous current drawn from the battery. When the boost converter shuts down, the instantaneous battery current drawn from the battery is immediately reduced a significant amount, thereby causing the input voltage to increase. The boost converter remains shut down until the microprocessor (e.g., the circuit U2 shown in FIG. 13A, described below), and/or other circuitry used with the boost converter, determine that it is time to turn the boost converter back ON. Once turned ON, the boost converter remains ON until, again, the input voltage drops to below 2.5 volts. This pattern continues, with the boost converter being ON for a short time, and OFF for a much longer time, thereby controlling and limiting the amount of current that can be drawn from the battery.

In the circuitry shown in FIG. 10, it is noted that the output voltage V_{OUT} generated by the boost converter circuit 200 is set by the reference voltage V_{REF} applied to the set point or feedback terminal of the boost converter circuit 200. For the configuration shown in FIG. 10, V_{REF} is proportional to the output voltage V_{OUT}, as determined by the resistor dividing network of R1 and R2.

The switches S_{P} and S_{R}, shown in FIG. 10 as part of the output circuit 230, are also controlled by the control circuit 220. These switches are selectively closed and opened to form the EA stimulation pulses applied to the load, R_{LOAD}. Before a stimulus pulse occurs, switch S_{R} is closed sufficiently long for the circuit side of coupling capacitor C_{C} to be charged to the output voltage, V_{OUT}. The tissue side of C_{C} is maintained at 0 volts by the electrode E2, which is maintained at ground reference. Then, for most of the time between stimulation pulses, both switches S_{R} and S_{P} are kept open, with a voltage approximately equal to the output voltage V_{OUT} appearing across the coupling capacitor C_{C}.

At the leading edge of a stimulus pulse, the switch S_{P} is closed, which immediately causes a negative voltage -V_{OUT} to appear across the load, R_{LOAD}, causing the voltage at the electrode E1 to also drop to approximately -V_{OUT}, thereby creating the leading edge of the stimulus pulse. This voltage starts to decay back to 0 volts as controlled by an RC (resistor-capacitance) time constant that is long compared with the desired pulse width. At the trailing edge of the pulse, before the voltage at the electrode E1 has decayed very much, the switch S_{P} is open and the switch S_{R} is closed. This action causes the voltage at the electrode E1 to immediately (relatively speaking) return to 0 volts, thereby defining the trailing edge of the pulse. With the switch S_{R} closed, the charge on the circuit side of the coupling capacitor C_{C} is allowed to charge back to V_{OUT} within a time period controlled by a time constant set by the values of capacitor C_{C} and resistor R3. When the circuit side of the coupling capacitor C_{C} has been charged back to V_{OUT}, then switch S_{R} is opened, and both switches S_{R} and S_{P} remain open until the next stimulus pulse is to be generated. Then the process repeats each time a stimulus pulse is to be applied across the load.

Thus, it is seen that in one embodiment of the electronic circuitry used within the IEAD 100, as shown in FIG. 10, a boost converter circuit 200 is employed which can be shut down with a control signal. The control signal is ideally a digital control signal generated by a control circuit 220 (which may be realized using a microprocessor or equivalent circuit). The control signal is applied to the low side (ground side) of the boost converter circuit 200 (identified as the "shutdown" terminal in FIG. 10). A capacitor C_{F} supplies instantaneous current for the short ON time that the control signal enables the boost converter circuit to operate. And, the capacitor CF is recharged from the battery during the relatively long OFF time when the control signal disables the boost converter circuit.

It is also seen that in a variation of the embodiment shown in FIG. 10, a boost converter circuit 200 is used that shuts itself down whenever the input voltage falls below a prescribed threshold, e.g., 2.5 V. The boost converter remains shut down until other circuitry used with the boost converter determines that it is time to turn the boost converter back ON, e.g., whenever the feedback signal indicates the output voltage V_{OUT} has fallen below a prescribed threshold, and/or whenever a prescribed period of time has elapsed since the last stimulus pulse was generated.

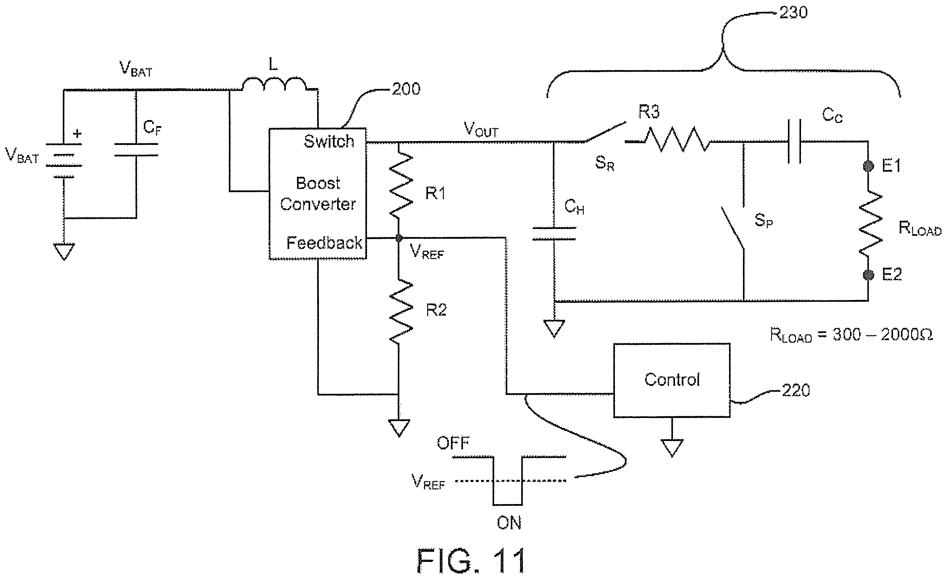

An alternate embodiment of the electronic circuitry that may be used within the IEAD 100 is shown in FIG. 11. The circuit shown in FIG. 11 is in most respects the same as the circuitry shown in FIG. 10. However, in this alternate embodiment shown in FIG. 11, the boost converter circuit 200 does not have a specific shut down input control. Rather, as seen in FIG. 11, the boost converter circuit is shut down by applying a control voltage to the feedback input of the boost converter circuit 200 that is higher than V_{REF}. When this happens, i.e., when the control voltage applied to the feedback input is greater than V_{REF}, the boost converter will stop switching and draws little or no current from the battery. The value of V_{REF} is typically a low enough voltage, such as a 1.2 V band-gap voltage, that a low level digital control signal can be used to disable the boost converter circuit. To enable the boost converter circuit, the control signal can be set to go to a high impedance, which effectively returns the node at the V_{REF} terminal to the voltage set by the resistor divider network formed from R1 and R2. Alternatively the control signal can be set to go to a voltage less than V_{REF}.

A low level digital control signal that performs this function of enabling (turning ON) or disabling (turning OFF) the boost converter circuit is depicted in FIG. 11 as being generated at the output of a control circuit 220. The signal line on which this control signal is present connects the output of the control circuit 220 with the V_{REF} node connected to the feedback input of the boost converter circuit. This control signal, as suggested by the waveform shown in FIG. 11, varies from a voltage greater than V_{REF}, thereby disabling or turning OFF the boost converter circuit, to a voltage less than V_{REF}, thereby enabling or turning the boost converter circuit ON.

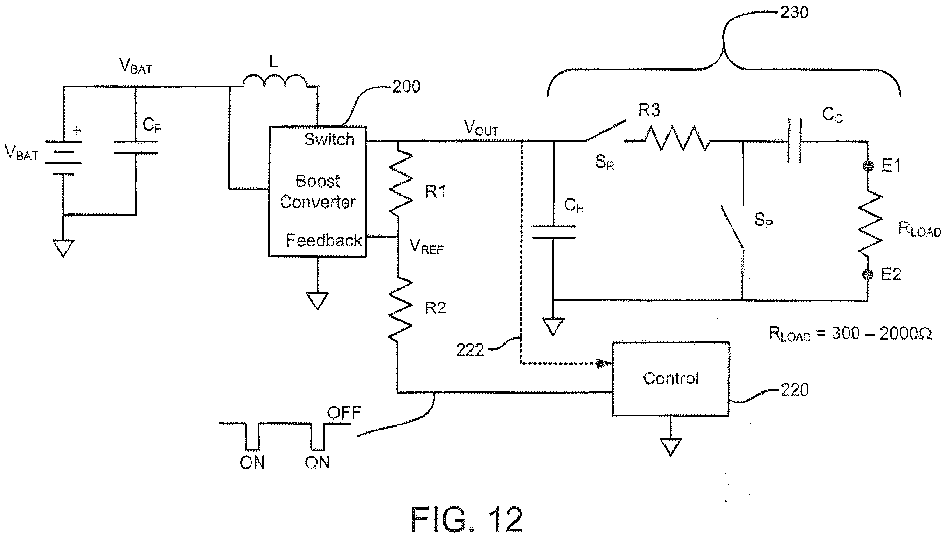

A refinement to the alternate embodiment shown in FIG. 11 is to use the control signal to drive the low side of R2 as shown in FIG. 12. That is, as shown in FIG. 12, the boost converter circuit 200 is shut down when the control signal is greater than V_{REF} and runs when the control signal is less than V_{REF}. A digital control signal can be used to perform this function by switching between ground and a voltage greater than V_{REF}. This has the additional possibility of delta-sigma modulation control of V_{OUT} if a measurement of the actual V_{OUT} is available for feedback, e.g., by using a signal line 222 coupled to the controller.

One preferred embodiment of the circuitry used in an implantable electroacupuncture device (IEAD) 100 that employs a digital control signal as taught herein is shown in the schematic diagram shown in FIG. 13A. In FIG. 13A, there are basically four integrated circuits (ICs) used as the main components. The IC U1 is a boost converter circuit, and performs the function of the boost converter circuit 200 described previously in connection with FIGS. 8, 9, 10, 11 and 12.

The IC U2 is a micro-controller IC and is used to perform the function of the control circuit 220 described previously in connection with FIGS. 10, 11 and 12. One preferred IC for this purpose is a MSP430G2452I micro-controller chip made by Texas Instruments. This chip includes 8 KB of Flash memory. Having some memory included with the micro-controller is important because it allows the parameters associated with a selected stimulation regimen to be defined and stored. One of the advantages of the EA device described herein is that it provides a stimulation regimen that can be defined with just 5 parameters, as taught below in connection with FIGS. 15A and 15B. This allows the programming features of the micro-controller to be carried out in a simple and straightforward manner.

The micro-controller U2 primarily performs the function of generating the digital signal that shuts down the boost converter to prevent too much instantaneous current from being drawn from the battery V_{BAT}. The micro-controller U2 also controls the generation of the stimulus pulses at the desired pulse width and frequency. It further keeps track of the time periods associated with a stimulation session, i.e., when a stimulation session begins and when it ends.

The micro-controller U2 also controls the amplitude of the stimulus pulse. This is done by adjusting the value of a current generated by a Programmable Current Source U3. In one embodiment, U3 is realized with a voltage controlled current source IC. In such a voltage controlled current source, the programmed current is set by a programmed voltage appearing across a fixed resistor R5, i.e., the voltage appearing at the "OUT" terminal of U3. This programmed voltage, in turn, is set by the voltage applied to the "SET" terminal of U3. That is, the programmed current source U3 sets the voltage at the "OUT" terminal to be equal to the voltage applied to the "SET" terminal. The programmed current that flows through the resistor R5 is then set by Ohms Law to be the voltage at the "set" terminal divided by R5. As the voltage at the "set" terminal changes, the current flowing through resistor R5 at the "OUT" terminal changes, and this current is essentially the same as the current pulled through the closed switch M1, which is essentially the same current flowing through the load R_{LOAD}. Hence, whatever current flows through resistor R5, as set by the voltage across resistor R5, is essentially the same current that flows through the load R_{LOAD}. Thus, as the micro-controller U2 sets the voltage at the "set" terminal of U3, on the signal line labeled "AMPSET", it controls what current flows through the load R_{LOAD}. In no event can the amplitude of the voltage pulse developed across the load R_{LOAD} exceed the voltage V_{OUT} developed by the boost converter less the voltage drops across the switches and current source.

The switches S_{R} and S_{P} described previously in connection with FIGS. 10, 11 and 12 are realized with transistor switches M1, M2, M3, M4, M5 and M6, each of which is controlled directly or indirectly by control signals generated by the micro-controller circuit U2. For the embodiment shown in FIG. 13A, these switches are controlled by two signals, one appearing on signal line 234, labeled PULSE, and the other appearing on signal line 236, labeled RCHG (which is an abbreviation for "recharge"). For the circuit configuration shown in FIG. 13A, the RCHG signal on signal line 236 is always the inverse of the PULSE signal appearing on signal line 234. This type of control does not allow both switch M1 and switch M2 to be open or closed at the same time. Rather, switch M1 is closed when switch M2 is open, and switch M2 is closed, when switch M1 is open. When switch M1 is closed, and switch M2 is open, the stimulus pulse appears across the load, R_{LOAD}, with the current flowing through the load, R_{LOAD}, being essentially equal to the current flowing through resistor R5. When the switch M1 is open, and switch M2 is closed, no stimulus pulse appears across the load, and the coupling capacitors C5 and C6 are recharged through the closed switch M2 and resistor R6 to the voltage V_{OUT} in anticipation of the next stimulus pulse.

The circuitry shown in FIG. 13A is only exemplary of one type of circuit that may be used to control the pulse width, amplitude, frequency, and duty cycle of stimulation pulses applied to the load, R_{LOAD}. Any type of circuit, or control, that allows stimulation pulses of a desired magnitude (measured in terms of pulse width, frequency and amplitude, where the amplitude may be measured in current or voltage) to be applied through the electrodes to the patient at the specified target tissue location at a desired duty cycle (stimulation session duration and frequency) may be used. However, for the circuitry to perform its intended function over a long period of time, e.g., years, using only a small energy source, e.g., a small coin-sized battery having a high battery impedance and a relatively low capacity, the circuitry must be properly managed and controlled to prevent excessive current draw from the battery.

It is also important that the circuitry used in the IEAD 100, e.g., the circuitry shown in FIGS. 10, 11, 12, 13A, or equivalents thereof, have some means for controlling the stimulation current that flows through the load, R_{LOAD}, which load may be characterized as the patient's tissue impedance at and around the target tissue location, e.g., acupoint, that is being stimulated. This tissue impedance, as shown in FIGS. 11 and 12, may typically vary from between about 300 ohms to 2000 ohms. Moreover, it not only varies from one patient to another, but it varies over time. Hence, there is a need to control the current that flows through this variable load, R_{LOAD}. One way of accomplishing this goal is to control the stimulation current, as opposed to the stimulation voltage, so that the same current will flow through the tissue load regardless of changes that may occur in the tissue impedance over time. The use of a voltage controlled current source U3, as shown in FIG. 13A, is one way to satisfy this need.

Still referring to FIG. 13A, a fourth IC U4 is connected to the micro-controller U2. For the embodiment shown in FIG. 13A, the IC U4 is an electromagnetic field sensor, and it allows the presence of an externally-generated (non-implanted) electromagnetic field to be sensed. An "electromagnetic" field, for purposes of this application includes magnetic fields, radio frequency (RF) fields, light fields, and the like. The electromagnetic sensor may take many forms, such as any wireless sensing element, e.g., a pickup coil or RF detector, a photon detector, a magnetic field detector, and the like. When a magnetic sensor is employed as the electromagnetic sensor U4, the magnetic field is generated using an External Control Device (ECD) 240 that communicates wirelessly, e.g., through the presence or absence of a magnetic field, with the magnetic sensor U4. (A magnetic field, or other type of field if a magnetic field is not used, is symbolically illustrated in FIG. 13A by the wavy line 242.) In its simplest form, the ECD 240 may simply be a magnet, and modulation of the magnetic field is achieved simply by placing or removing the magnet next to or away from the IEAD. When other types of sensors (nonmagnetic) are employed, the ECD 240 generates the appropriate signal or field to be sensed by the sensor that is used.

Use of the ECD 240 provides a way for the patient, or medical personnel, to control the IEAD 100 after it has been implanted (or before it is implanted) with some simple commands, e.g., turn the IEAD ON, turn the IEAD OFF, increase the amplitude of the stimulation pulses by one increment, decrease the amplitude of the stimulation pulses by one increment, and the like. A simple coding scheme may be used to differentiate one command from another. For example, one coding scheme is time-based. That is, a first command is communicated by holding a magnet near the IEAD 100, and hence near the magnetic sensor U4 contained within the IEAD 100, for differing lengths of time. If, for example, a magnet is held over the IEAD for at least 2 seconds, but no more than 7 seconds, a first command is communicated. If a magnet is held over the IEAD for at least 11 seconds, but no more than 18 seconds, a second command is communicated, and so forth.

Another coding scheme that could be used is a sequence-based coding scheme. That is, application of 3 magnetic pulses may be used to signal one external command, if the sequence is repeated 3 times. A sequence of 2 magnetic pulses, repeated twice, may be used to signal another external command. A sequence of one magnetic pulse, followed by a sequence of two magnetic pulses, followed by a sequence of three magnetic pulses, may be used to signal yet another external command.

Other simple coding schemes may also be used, such as the letters AA, RR, HO, BT, KS using international Morse code. That is, the Morse code symbols for the letter "A" are dot dash, where a dot is a short magnetic pulse, and a dash is a long magnetic pulse. Thus, to send the letter A to the IEAD 100 using an external magnet, the user would hold the magnet over the area where the IEAD 100 is implanted for a short period of time, e.g., one second or less, followed by holding the magnet over the IEAD for a long period of time, e.g., more than one second.

More sophisticated magnetic coding schemes may be used to communicate to the micro-controller chip U2 the operating parameters of the IEAD 100. For example, using an electromagnet controlled by a computer, the pulse width, frequency, and amplitude of the EA stimulation pulses used during each stimulation session may be pre-set. Also, the frequency of the stimulation sessions can be pre-set. Additionally, a master reset signal can be sent to the device in order to re-set these parameters to default values. These same operating parameters and commands may be re-sent at any time to the IEAD 100 during its useful lifetime should changes in the parameters be desired or needed.

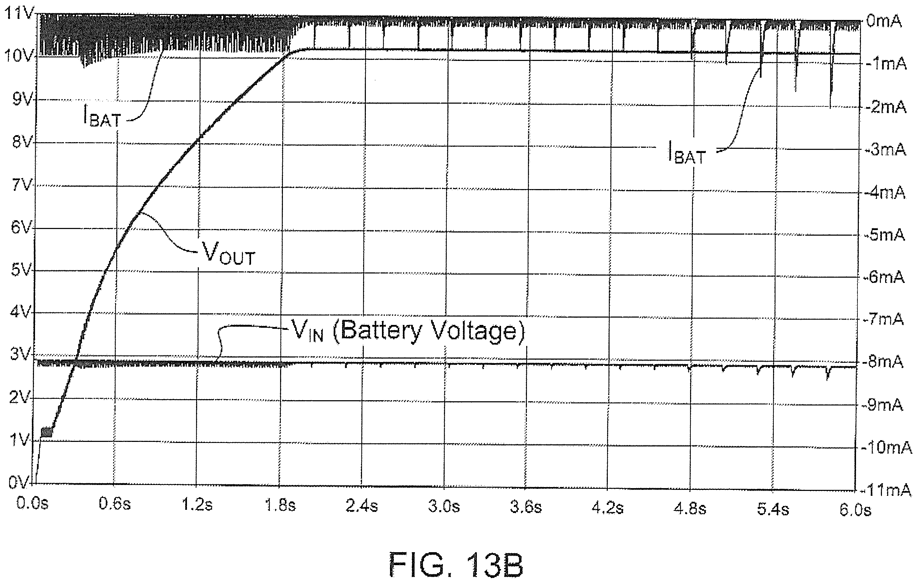

The current and voltage waveforms associated with the operation of the IEAD circuitry of FIG. 13A are shown in FIG. 13B. In FIG. 13B, the horizontal axis is time, the left vertical axis is voltage, and the right vertical axis is current. The battery in this example has 160 Ohms of internal impedance.

Referring to FIGS. 13A and 13B, during startup, the boost converter ON time is approximately 30 microseconds applied every 7.8 milliseconds. This is sufficient to ramp the output voltage V_{OUT} up to over 10 V within 2 seconds while drawing no more than about 1 mA from the battery and inducing only 150 mV of input voltage ripple.

The electroacupuncture (EA) simulation pulses resulting from operation of the circuit of FIG. 13A have a width of 0.5 milliseconds and increase in amplitude from approximately 1 mA in the first pulse to approximately 15 mA in the last pulse. The instantaneous current drawn from the battery is less than 2 mA for the EA pulses and the drop in battery voltage is less than approximately 300 mV. The boost converter is enabled (turned ON) only during the instantaneous output current surges associated with the 0.5 milliseconds wide EA pulses.

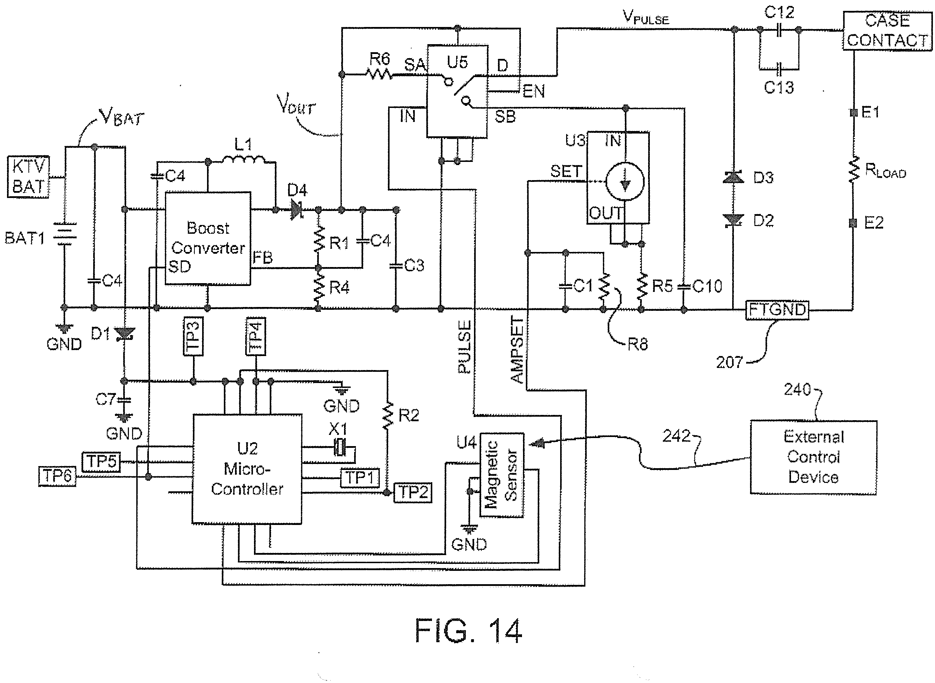

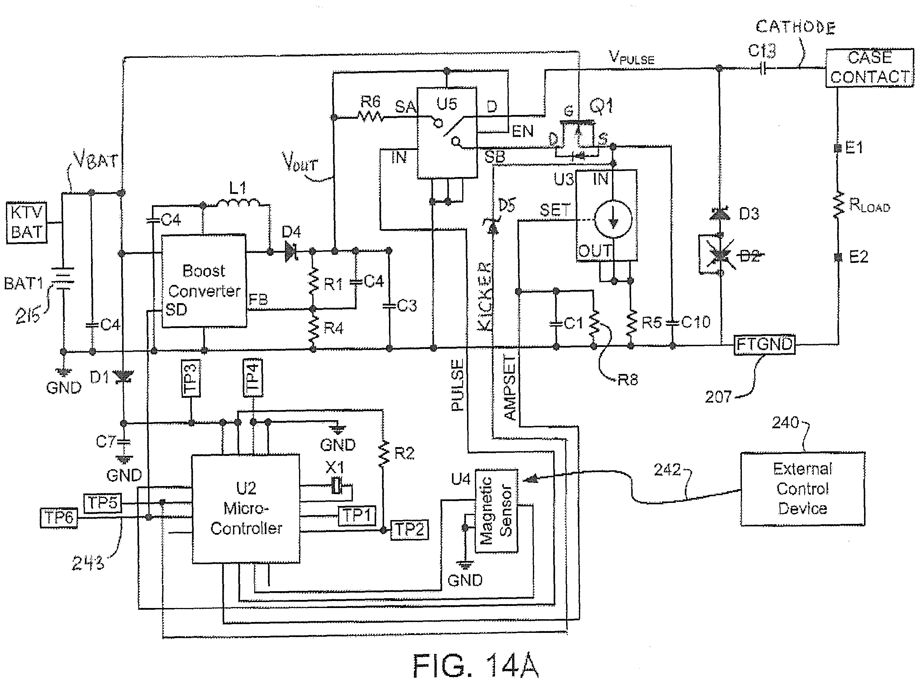

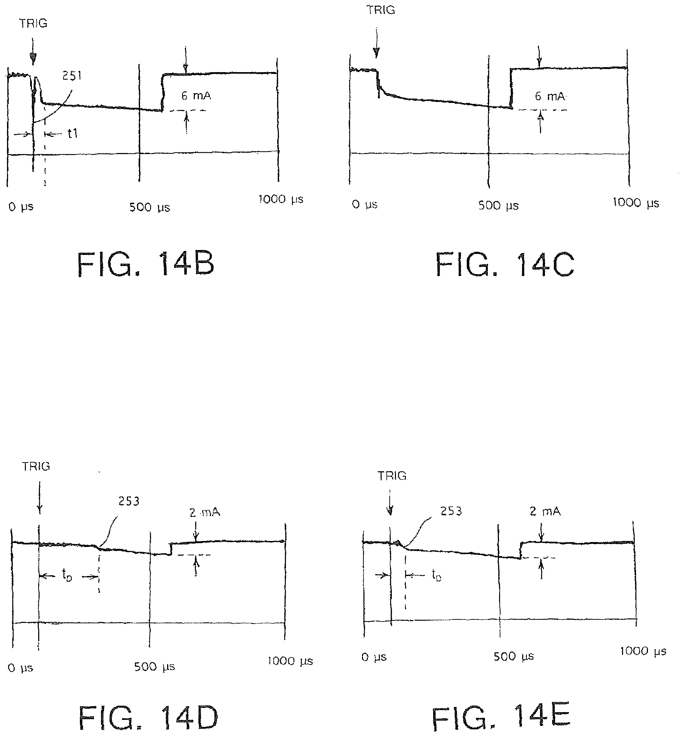

Another preferred embodiment of the circuitry used in an implantable electroacupuncture (EA) device 100 (also referred to as an IEAD 100) that employs a digital control signal as taught herein is shown in the schematic diagram of FIG. 14. The circuit shown in FIG. 14 is, in most respects, very similar to the circuit described previously in connection with FIG. 13A. What is new in FIG. 14 is the inclusion of a Schottky diode D4 at the output terminal LX of the boost convertor U1 and the inclusion of a fifth integrated circuit (IC) U5 that essentially performs the same function as the switches M1-M6 shown in FIG. 13A.

The Schottky diode D4 helps isolate the output voltage V_{OUT} generated by the boost converter circuit U1. This is important in applications where the boost converter circuit U1 is selected and operated to provide an output voltage V_{OUT} that is four or five times as great as the battery voltage, V_{BAT}. For example, in the embodiment for which the circuit of FIG. 14 is designed, the output voltage V_{OUT} is designed to be nominally 15 volts using a battery that has a nominal battery voltage of only 3 volts. (In contrast, the embodiment shown in FIG. 13A is designed to provide an output voltage that is nominally 10-12 volts, using a battery having a nominal output voltage of 3 volts.)

The inclusion of the fifth IC U5 in the circuit shown in FIG. 14 is used, as indicated, to perform the function of a switch. The other ICs shown in FIG. 14, U1 (boost converter), U2 (micro-controller), U3 (voltage controlled programmable current source) and U4 (electromagnetic sensor) are basically the same as the IC's U1, U2, U3 and U4 described previously in connection with FIG. 13A.

The IC U5 shown in FIG. 14 functions as a single pole/double throw (SPDT) switch. Numerous commercially-available ICs may be used for this function. For example, an ADG1419 IC, available from Analog Devices Incorporated (ADI) may be used. In such IC U5, the terminal "D" functions as the common terminal of the switch, and the terminals "SA" and "SB" function as the selected output terminal of the switch. The terminals "IN" and "EN" are control terminals to control the position of the switch. Thus, when there is a signal present on the PULSE line, which is connected to the "IN" terminal of U5, the SPDT switch U5 connects the "D" terminal to the "SB" terminal, and the SPDT switch U5 effectively connects the cathode electrode E1 to the programmable current source U3. This connection thus causes the programmed current, set by the control voltage AMPSET applied to the SET terminal of the programmable current source U3, to flow through resistor R5, which in turn causes essentially the same current to flow through the load, R_{LOAD}, present between the electrodes E1 and E2. When a signal is not present on the PULSE line, the SPDT switch U5 effectively connects the cathode electrode E1 to the resistor R6, which allows the coupling capacitors C12 and C13 to recharge back to the voltage V_{OUT} provided by the boost converter circuit U2.

Yet another preferred embodiment of the circuitry used in an implantable electroacupuncture device (IEAD) 100 that employs an ON-OFF approach to duty-cycle modulate the boost converter as a tool for limiting the amount of instantaneous battery current drawn from the high impedance battery 215 is shown in the schematic diagram of FIG. 14A. The circuit shown in FIG. 14A is, in most respects, very similar to, or the same as, the circuit described previously in connection with FIG. 14 or FIG. 13A, and that description will not be repeated here. What is new in FIG. 14A are the addition of elements and features that address additional issues associated with the operation of an IEAD 100.

One feature included in the circuitry of FIG. 14A, which is described briefly above in connection with the description of FIG. 10, is that the boost converter circuit U1 is modulated ON and OFF using digital control generated within the boost converter circuit U1 itself. In accordance with this variation, the boost converter circuit 200 shuts itself down whenever the battery voltage falls below a predetermined level above that required by the remaining circuitry. For example, in the embodiment shown in FIG. 14A, the boost converter circuit U1 is realized using a MAX8570 boost converter IC, commercially available from Maxim, or equivalents thereof. This particular boost converter IC shuts down when the applied voltage, V_{BAT}, falls below 2.5 V. Advantageously, a battery voltage of 2.5 volts is still high enough to ensure the microcontroller IC U2, and other circuitry associated with the operation of the IEAD 100, remain operational.

Thus, in operation, as soon as the battery voltage drops below 2.5 volts, the boost converter circuit U1 shuts down, thereby limiting the instantaneous current drawn from the battery. When the boost converter U1 shuts down, the instantaneous battery current drawn from the battery is immediately reduced a significant amount, thereby causing the battery voltage V_{BAT} to increase.

As the battery voltage V_{BAT} increases, the boost converter circuit U1 remains shut down until the microcontroller U2 determines that it is time to turn the boost converter back ON. This turn ON typically occurs in one of two ways: (1) just prior to the delivery of the next stimulus pulse, a turn ON signal may be applied to the Shutdown ("SD") terminal, signal line 243, of the boost converter circuit U1; or (2) as soon as the battery voltage, V_{BAT}, has increased a sufficient amount, as sensed at the feedback terminal FB of the boost converter circuit U1, the circuits within the boost converter circuit U1 are automatically turned back ON, allowing the output voltage V_{OUT} to build up to a voltage level needed by the switch circuit U5 and the current source circuit U3 to generate an output stimulus pulse of the desired amplitude when the next PULSE signal is applied to the IN terminal of the switch U5 by the microcontroller U2.

Once turned ON, the boost converter remains ON until, again, the input voltage drops below 2.5 volts. This pattern continues, with the boost converter being ON for a short time, and OFF for a much longer time (typically, the duty cycle associated with this ON/OFF operation of the boost converter circuit U1 is no greater than about 0.01), thereby controlling and limiting the amount of current that is drawn from the battery. This ON/OFF action of U1 assures that the battery voltage, V_{BAT}, always remains sufficiently high to permit operation of all the critical circuits of the IEAD 100 (principally the circuits of the microcontroller U2), except the boost converter circuit U1.

In a preferred implementation, the microcontroller circuit U2 used in FIG. 14A is an MSP430G2452IRSA 16 microcontroller, commercially available from Texas Instruments, or equivalent microcontroller The current source circuit U3 comprises a LT3092 programmable current source commercially available form Linear Technology, or equivalents thereof. The sensor circuit U4 comprises an AS-M15SA-R magnetic sensor, commercially available from Murata, or equivalents thereof. And, the switch circuit U5 comprises an ADG1419BCPZ single pole double throw analog switch commercially available from Analog Devices, or equivalents thereof.

Another feature or enhancement provided by the circuit implementation depicted in FIG. 14A relates to removing, or at least minimizing, some undesirable leading edge transients that would otherwise be seen in the output stimulus pulses generated by the circuitry of FIG. 14A. The solution to remove or mitigate the occurrence of such leading edge transients is to insert an N-MOSFET transistor switch Q1 at the input terminal, IN, of the programmable current source circuit U3. This switch Q1 acts as a "cascode" stage that maintains a more constant voltage across the current source U3 as the output current and/or load resistance changes. The gate (G) terminal of the switch Q1 is driven by the battery voltage, V_{BAT}, which means the voltage at the source terminal (S) of switch Q1, which is connected to the IN terminal of the current source U3, is limited to roughly V_{BAT} - V_{GS}, where V_{GS} is the threshold voltage across the gate (G)-source (S) terminals of Q1.

Use of this N-MOSFET switch Q1 as depicted in FIG. 14A advantageously reduces the transient leading edge of the stimulus pulse because the capacitance looking into Q1 is much less than is seen when looking into the current source circuit U3 because of the Miller effect. That is, there is considerable loop gain in the operation of the U3 current source circuit to servo the current. This loop gain directly scales the input capacitance so that there is a much larger leading edge spike on the pulse. This in turn causes a 30 to 40 microsecond transient at the leading edge of the current pulse as the current source U3 recovers current regulation.

An example of this leading edge transient is illustrated in the timing waveform diagram of FIG. 14B. In FIG. 14B (as well as in FIGS. 14C, 14D and 14E, which all show similar timing waveform diagrams), the horizontal axis is time and the vertical axis is voltage, which (assuming a resistive load of 600 ohms) may readily be converted to current, as has been done in these figures. The stimulus pulse begins at a trigger location near the left edge of the waveform, labeled TRIG. As seen in FIG. 14B, immediately after the trigger point, which should mark the beginning or leading edge of the stimulus pulse, an initial spike 251 occurs that has a magnitude on the order of twice the amplitude of the stimulus pulse. This spike 251 shoots down (as the waveform is oriented in the figures) and then shoots back up, and eventually, after a delay of t1 microseconds, becomes the leading edge of the pulse. The delay t1 is about 30-40 microseconds, which means that the leading edge of the stimulus pulse is delayed 30-40 microseconds. Having a leading edge delay of this magnitude is not a desirable result.

Next, with the cascode stage (comprising the switch Q1) connected to the input terminal, IN, of the current source U3, the stimulus pulse is again illustrated. Because the cascode stage significantly reduces the input capacitance looking into the drain (D) terminal of the switch Q1, the leading edge transient is significantly reduced, as illustrated in the timing waveform diagram of FIG. 14C. As seen in FIG. 14C, the leading edge transient has all but disappeared, and the delay t1 between the trigger point and the leading edge of the stimulus pulse is negligible.

Another feature or enhancement provided by the circuitry of FIG. 14A is to address a delay that is seen when starting up the programmable current source U3 at low pulse amplitudes, (e.g., less than about 3 mA). A typical current stimulus output for the IEAD is on the order of 15-25 mA. When a much smaller amplitude current stimulus is used, e.g., 1.5-3 mA, the control signal that defines this smaller amplitude pulse is significantly less than the one used to define the more typical stimulus amplitudes of 15-25 mA. Such a small control signal lengthens the delay, t_{D}, between the trigger point, TRIG, and the leading edge 253 of the stimulus pulse. FIG. 14D illustrates this long delay, t_{D}, which is on the order of 200 microseconds.

To address the problem illustrated in the waveform diagram of FIG. 14D, a Schottky diode D5 is connected in the circuit of FIG. 14A from an output port on the microcontroller circuit U2 to the input port, IN, of the current source circuit U3. In a preferred implementation of the circuit of FIG. 14A, this Schottky diode D5 is realized using a BAT54XV2DKR diode, commercially available from Fairchild Semiconductor, or equivalents thereof. This diode is used to warm-up or "kick start" the circuit U3 when the pulse amplitude is low so that there is less of a delay, t_{D}, before current is regulated at the start of the pulse. Since the cascode stage Q1 keeps the drop across U3 low, U3 can be driven directly from the microcontroller U2 at the start of the pulse without significantly changing the pulse characteristics (e.g., amplitude or timing) in such a way that the delay, t_{D}, before current is regulated at the start of the pulse can be reduced.

FIG. 14E illustrates the timing waveform diagram achieved using the circuit of FIG. 14A with the diode D5 inserted so as to allow the microcontroller U2 to directly drive, or "kick start", the current source circuit U3 at the start of the pulse. As seen in FIG. 14E, the delay, t_{D}, realized with the "kick start" has been significantly reduced from what it was without the "kick start" (as shown in FIG. 14D), e.g., from about 200 microseconds to about 40 microseconds, or less. Thus, this "kick start" feature shortens the undesired delay, t_{D}, by at least a factor of about 5.

An additional feature provided by the circuitry of FIG. 14A addresses a concern regarding EMI (electro*ma*gnetic *i*nterference). EMI can occur, for example, during electrocautery and/or external defibrillation. Should any of the circuit elements used within the IEAD 100, such as the analog switch U5, have a transient voltage exceeding approximately 0.3 V appear on its pins (which transient voltage could easily occur if the IEAD is subjected to uncontrolled EMI), then the IC could be damaged. To prevent such possible EMI damage, the output voltage pulse, appearing on the signal line labeled V_{PULSE}, is clamped to ground through the forward bias direction of the zenor diode D3. In contrast, in the circuits shown in FIGS. 13A and 14, there are two zenor diodes, D2 and D3, connected back to back, to limit the voltage appearing on the V_{PULSE} line to voltages no greater than the zenor diode voltage in either direction. As seen in FIG. 14A, diode D2 has been replaced with a short, e.g., diode D2 is removed and replaced with a wire, thereby clamping the voltage that can appear on the output voltage line -the signal line where V_{PULSE} appears-- in one polarity direction to no greater than the forward voltage drop across the diode D3.

As is evident from the waveforms depicted in FIGS. 14B, 14C, 14D and 14E, the basic current stimulus waveform is not a square wave, with a "flat top", (or, in the case of a negative current waveform, with a "flat bottom") as depicted in most simplified waveform diagrams (see, e.g., FIG. 15A). Rather, the current stimulus waveforms shown in FIGS. 14B, 14C, 14D and 14E have what the inventors refer to as a reverse trapezoidal shape. That is, the current waveforms start at a first value, at the leading edge of the pulse, and gradually ramp to a second, larger, value at the trailing edge of the pulse (i.e., the current increases during the pulse). For a negative-going pulse, as is shown in these figures, the ramp slopes downward, but this corresponds to the amplitude of the pulse getting larger.

This pulse shape -a reverse trapezoidal shape-- for the current stimulus pulse is by design. That is, the inventors want the current to increase during the pulse because such shape is believed to be more selective for the recruitment of smaller fiber diameter tissue and nerves, and thus has the potential to be more effective in achieving its intended goal of activating desired tissue at the target tissue location.

The reverse trapezoidal stimulus pulse shape is illustrated in more detail in FIG. 15, as is one manner for achieving it. Shown on the right side of FIG. 15 is a sketch of a reverse trapezoidal pulse. (Note, it is referred to as a "reverse trapezoidal" pulse because the current, or waveform, gets larger or increases during the pulse. This is in contrast to a conventional voltage regulated pulse, which is "trapezoidal", but in the other direction, i.e., the current decreases during the pulse.) As seen in FIG. 15, the reverse trapezoidal pulse has a duration T1, but the magnitude (amplitude) of the current during the pulse increases from a first value at the leading edge of the pulse to a second value at the trailing edge of the pulse. The increase in current from the leading edge of the pulse to the trailing edge is a value Ap. The average amplitude of the pulse during the pulse time T1 is a value A1, which is typically measured at a time T_{M}, which is about in the middle of the pulse. That is, T_{M} = ½T1.

Also shown in FIG. 15, on the left side, is the circuitry that is used to create the reverse trapezoidal waveform. This circuitry is part of the circuitry shown, e.g., in FIG. 14A, and includes a capacitor C1 in parallel with a large resistor R8 (270 KΩ) connected to the "set" terminal of the programmable current source U3. The "AMPSET" signal, generated by the micro-controller circuit U2 to set the amplitude A1 of the current stimulus pulse to be generated, is applied to the "set" terminal of U3. When enabled by the AMPSET signal, the capacitor C1 starts to charge up during the pulse at a rate of approximately 10 µA (which comes from the "set" pin of U3, i.e., from the circuitry inside of U3). For C1 = 0.1 microfarads, this turns out to be 100 mV/ms, or 50 mV for a pulse having a pulse duration or width (T1) of 0.5 ms. Since the pulse current is approximately equal to V_{SET}/R5, the pulse current will increase by 50mV/R5. Or, where R5 is 22 ohms, this increase in current turns out to be 50mV/22 = 2.27 mA at the end of the 0.5 ms pulse. This increase is essentially fixed regardless of the programmed pulse amplitude.

While the circuitry described above performs the intended function of causing the current stimulus pulse to have a reverse trapezoidal shape in a simple and straightforward manner, it should be noted that there are other circuits and techniques that could also be used to achieve this same result. Moreover, it would be possible to directly control the shape of the V_{SET} signal during the pulse duration in order to create any desired stimulus pulse shape.

As shown in embodiment of the IEAD shown in FIG. 14A, the stimulation circuitry uses a micro-controller integrated circuit (IC) U2 which generates all of the operating control signals needed to guide other circuits, including the Boost Converter circuit IC U1, to generate the desired stream of stimulation pulses. These other circuits include a programmable current source IC U3, an analog switch U5, and a magnetic sensor U4. As can be seen in FIG. 14A, the micro-controller circuit U2 is driven by a clock circuit that includes a crystal oscillator to provide a very stable frequency reference. However, when the stimulus pulses are not being generated - which is most of the time given the very low duty cycle of operation, e.g., T3/T4 is less than 0.05- - the micro-controller U2 is able to go into a very low power sleep state, thereby conserving power.

In order for the present invention to provide accurate chronotherapeutics (i.e., the delivery of stimulation sessions having very precise stimulation parameters at very precise times), it would be desirable to use a crystal time base. In the existing micro-controller U2 design, however, the crystal clock circuit does not provide an accurate time base; rather, all it provides is a steady clock signal that can be counted using simple counter circuits. A crystal time base, on the other hand, could accurately perform all the functions of a rather sophisticated stop watch, including keeping track of multiple time bases.

A crystal time base (operating all the time - which it would need to do to provide accurate chronotherapeutics) would roughly double the battery current between therapy sessions, thereby taking the nominal longevity of the implantable electroacupuncture device (IEAD) down roughly from 3 years to 2 years.

Reducing the longevity of the IEAD by a factor of 1/3 is not viewed as an acceptable tradeoff to provide accurate chronotherapeutics. Hence, what is needed is a design, or alternate approach, whereby an accurate time base could be provided without sacrificing a significant loss in longevity of the IEAD.

One way to accomplish this desired result is to add another small IC to the circuits of the IEAD that functions as a real time clock (RTC). Such RTC may be realized from a very small device (3.2 x 1.5 mm) that runs on 360 nanoAmps (nA) of current. Such device, referred to as a Real Time Clock Module is commercially available from Micro Crystal AG, of Grenchen, Switzerland, as part number RV-4162-C7.

A schematic diagram of an IEAD design that uses such an RTC Module to replace the crystal time base presently used with the micro-controller U2 is shown in FIG. 15C. In most respects, the circuit shown in FIG. 15C is the same as the circuit shown in FIG. 14A. The one key difference between the IEAD circuit of FIG. 15A and the IEAD circuit of FIG. 14A, is the insertion of a RTC module U6. As seen in FIG. 15C, the RTC module U6 is connected to the micro-controller circuit U2 and replaces the previously-used external crystal oscillator. The RTC module U6 is able to be set to wake up the micro-controller circuit U2 when needed, and/or put the micro-controller U2 in a shut-down (sleep) state when not needed. Advantageously, the shutdown mode is even a lower power state than is achieved with the sleep state controlled by the previously-used external crystal oscillator.

From the above description, it is seen that an implantable IEAD 100 is provided that uses a digital control signal to duty-cycle limit the instantaneous current drawn from the battery by a boost converter. Three different exemplary configurations (FIGS 10, 11 and 12) are taught for achieving this desired result, and four exemplary circuit designs, or implementations, have been presented that may be used to realize the desired configurations (FIGS. 13A, 14, 14A and 15C). One implementation (FIG. 15C), in addition to including all the enhancements incrementally added to the base implementation circuit of FIG. 13A (to address, e.g., problems of slow starts, delays, and needed circuit isolation, and to add improvements such as better switching, reverse trapezoidal stimulation wave shapes and EMI protection, teaches the use of a real time clock module to provide a crystal time base to facilitate the use of chronotherapeutics. One configuration (FIG. 12) teaches the additional capability to delta-sigma modulate the boost converter output voltage.

Delta-sigma modulation is well described in the art. Basically, it is a method for encoding analog signals into digital signals or higher-resolution digital signals into lower-resolution digital signals. The conversion is done using error feedback, where the difference between the two signals is measured and used to improve the conversion. The low-resolution signal typically changes more quickly than the high-resolution signal and it can be filtered to recover the high resolution signal with little or no loss of fidelity. Delta-sigma modulation has found increasing use in modern electronic components such as converters, frequency synthesizers, switched-mode power supplies and motor controllers. See, e.g., Wikipedia, *Delta-sigma modulation.*

### Use and Operation

With the implantable electroacupuncture device (IEAD) 100 in hand, the IEAD 100 may be used most effectively to treat a specified disease or medical condition of the patient by first pre-setting stimulation parameters that the device will use during a stimulation session. FIGS. 15A and 15B show timing waveform diagrams illustrating the EA stimulation parameters used by the IEAD to generate EA stimulation pulses. As seen in FIG. 15A, there are basically four parameters associated with a stimulation session. The time T1 defines the duration (or pulse width) of a stimulus pulse. The time T2 defines the time between the start of one stimulus pulse and the start of the next stimulus pulse. The time T2 thus defines the period associated with the frequency of the stimulus pulses. The frequency of the stimulation pulses is equal to 1/T2. The ratio of T1/T2 is typically quite low, e.g., less than 0.01, but may, in some instances, be as much as 0.03. The duration of a stimulation session is dictated or defined by the time period T3. The amplitude of the stimulation pulses is defined by the amplitude A1. This amplitude may be expressed in either voltage or current.

FIG. 15B illustrates the manner in which the stimulation sessions are administered in accordance with a specified stimulation regimen. FIG. 15B shows several stimulation sessions of duration T3, and how often the stimulation sessions occur. The stimulation regimen thus includes a time period T4 which sets the time period from the start of one stimulation session to the start of the next stimulation session. T4 thus is the period of the stimulation session frequency, and the stimulation session frequency is equal to 1/T4.

In order to allow the applied stimulation to achieve its desired effect on the body tissue at the selected target stimulation site, the period of the stimulation session T4 may be varied when the stimulation sessions are first applied. This can be achieved by employing a simple algorithm within the circuitry of the EA device that changes the value of T4 in an appropriate manner. For example, at start up, the period T4 may be set to a minimum value, T4(min). Then, as time goes on, the value of T4 may be gradually increased until a desired value of T4, T4(final), is reached.

By way of example, if T4(min) is 1 day, and T4(final) is 7 days, the value of T4 may vary as follows once the stimulation sessions begin: T4=1 day for the duration between the first and second stimulation sessions, then 2 days for the duration between the second and third stimulation sessions, then 4 days for the duration between the third and fourth stimulation sessions, and then finally 7 days for the duration between all subsequent stimulation sessions after the fourth stimulation session.

Rather than increasing the value of T4 from a minimum value to a maximum value using a simple doubling algorithm, as described in the previous paragraph, an enhancement is to use a table that defines session durations and intervals whereby the automatic session interval can be shorter for the first week or so. For example, T3 is 30 minutes, the first 30 minute session could be delivered after 1 day. The second 30 minute session could be delivered after 2 days. The third 30 minute session could be delivered after 4 days. Finally, the 4^{th} 30 minute session could be delivered for all subsequent sessions after 7 days.

If a triggered session is delivered completely, it advances the therapy schedule to the next table entry.

Another enhancement is that the initial set amplitude only takes effect if the subsequent triggered session is completely delivered. If the first session is aborted by a magnet application, the device reverts to a Shelf Mode. In this way, the first session is always a triggered session that occurs in the clinician setting.

Finally, the amplitude and place in the session table are saved in non-volatile memory when they change. This avoids a resetting of the therapy schedule and need to reprogram the amplitude in the event of a device reset.

By way of example, one preferred set of parameters to use to define a stimulation regimen is:
T1 = 0.5 milliseconds
T2 = 1000 milliseconds
T3 = 30 minutes
T4 = 7 days (10,080 minutes)
A1 = 20 volts (across 1 KΩ), or 20 milliAmperes (mA)

Another example of a preferred set of parameters, with acceptable ranges for each parameter, for treating a particular condition or disease is as follows:
T1 = 0.1 to 2.0 milliseconds (ms)
T2 = 67 to 1000 ms (15 Hz to 1 Hz)
T3 = 10 to 60 minutes
T4 = 1,440 to 20,160 minutes (1 day to 2 weeks)
A1 = 1 to 25 mA

It is to be emphasized that the values shown above for the stimulation regimen and ranges of stimulation parameters for use within the stimulation regimen are only exemplary. Other stimulation regimens that could be used, and the ranges of values that could be used for each of these parameters, are as defined in the claims.

It is also emphasized that the ranges of values presented in the claims for the parameters used with the invention have been selected after many months of careful research and study, and are *not* arbitrary. For example, the ratio of T3/T4, which sets the duty cycle, has been carefully selected to be very low, e.g., no more than 0.05. Maintaining a low duty cycle of this magnitude represents a significant change over what others have attempted in the implantable stimulator art. Not only does a very low duty cycle allow the battery itself to be small (coin-cell size), which in turn allows the IEAD housing to be very small, which makes the IEAD ideally suited for being used without leads, thereby making it relatively easy to implant at the desired stimulation site (e.g., acupoint), but it also limits the frequency and duration of stimulation sessions.

Limiting the frequency and duration of the stimulation sessions is a key aspect of Applicant's invention because it recognizes that some treatments are best done slowly and methodically, over time, rather than quickly and harshly using large doses of stimulation (or other treatments) aimed at forcing a rapid change in the patient's condition. Moreover, applying treatments slowly and methodically is more in keeping with traditional acupuncture methods (which, as indicated previously, are based on over 2500 years of experience). In addition, this slow and methodical conditioning is consistent with the time scale for remodeling of the central nervous system needed to produce a sustained therapeutic effect. Thus, Applicant has based its treatment regimen on the slow-and-methodical approach, as opposed to the immediate-and-forced approach adopted by many, if not most, prior art implantable electrical stimulators.

Once the stimulation regimen has been defined and the parameters associated with it have been pre-set into the memory of the micro-controller circuit U2, the IEAD 100 is ready to be implanted. Implantation is usually a simple procedure, and is described above in connection, e.g., with the description of FIGS. 1A and 1B.

After implantation, the IEAD must be turned ON, and otherwise controlled, so that the desired stimulation regimen or stimulation paradigm may be carried out. In one preferred embodiment, control of the IEAD after implantation, as well as any time after the housing of the IEAD has been hermetically sealed, is performed as shown in the state diagram of FIG. 16. Each circle shown in FIG. 16 represents an operating "state" of the micro-controller U2 (FIGS. 13A, 14, 14A or 15C). As seen in FIG. 16, the controller U2 only operates in one of six states: (1) a "Set Amplitude" state, (2) a "Shelf Mode" state, (3) a "Triggered Session" state, (4) a "Sleep" state, (5) an "OFF" state, and an (6) "Automatic Session" state. The "Automatic Session" state is the state that automatically carries out the stimulation regimen using the pre-programmed parameters that define the stimulation regimen.

Shelf Mode is a low power state in which the IEAD is placed prior to shipment. After implant, commands are made through magnet application. Magnet application means an external magnet, typically a small hand-held cylindrical magnet, is placed over the location where the IEAD has been implanted. With a magnet in that location, the magnetic sensor U4 senses the presence of the magnet and notifies the controller U2 of the magnet's presence.

From the "Shelf Mode" state, a magnet application for 10 seconds (M.10s) puts the IEAD in the "Set Amplitude" state. While in the "Set Amplitude" state, the stimulation starts running by generating pulses at zero amplitude, incrementing every five seconds until the patient indicates that a comfortable level has been reached. At that time, the magnet is removed to set the amplitude.

If the magnet is removed and the amplitude is non-zero (*M̅* ∧ *A*), the device continues into the 'Triggered Session" so the patient receives the initial therapy. If the magnet is removed during "Set Amplitude" while the amplitude is zero (*M̅* ∧ *A̅*), the device returns to the Shelf Mode.

The Triggered Session ends and stimulation stops after the session time (T_{S}) has elapsed and the device enters the "Sleep" state. If a magnet is applied during a Triggered Session (M), the session aborts to the "OFF" state. If the magnet remains held on for 10 seconds (M.10s) while in the "OFF" state, the "Set Amplitude" state is entered with the stimulation level starting from zero amplitude as described.

If the magnet is removed (*M̅*) within 10 seconds while in the OFF state, the device enters the Sleep state. From the Sleep state, the device automatically enters the Automatic Session state when the session interval time has expired (T_{I}). The Automatic Session delivers stimulation pulses at the set amplitude (which many vary, e.g., ramp up from zero, depending on the stimulus regimen) for the session time (T_{S}) and the device returns to the Sleep state. In this embodiment, the magnet has no effect once the Automatic Session starts so that the full therapy session is delivered.

While in the Sleep state, if a magnet has not been applied in the last 30 seconds (D) and a magnet is applied for a window between 20-25 seconds and then removed (M.20:25s), a Triggered Session is started. If the magnet window is missed (i.e. magnet removed too soon or too late), the 30 second de-bounce period (D) is started. When de-bounce is active, no magnet must be detected for 30 seconds before a Triggered Session can be initiated.

The session interval timer runs while the device is in Sleep state. The session interval timer is initialized when the device is woken up from Shelf Mode and is reset after each session is completely delivered. Thus abort of a triggered session by magnet application will not reset the timer, the Triggered Session must be completely delivered.

The circuitry that sets the various states shown in FIG. 16 as a function of externally-generated magnetic control commands, or other externally-generated command signals, is the micro-controller U2 (FIG. 14), the processor U2 (FIG. 13A), or the control circuit 220 (FIGS. 10, 11 and 12). Such processor-type circuits are programmable circuits that operate as directed by a program. The program is often referred to as "code", or a sequence of steps that the processor circuit follows. The "code" can take many forms, and be written in many different languages and formats, known to those of skill in the art. It is submitted that a person of skill in the art, given the states that the IEAD may assume, and the control commands applied to change from one state to another, as presented in the state diagram of FIG. 16, could write appropriate code for the micro-controller U2 (FIGS. 13A, 14, 14A or 15C) for controlling the states of the IEAD as shown in FIG. 16.

In the preceding description, various exemplary embodiments have been described with reference to the accompanying drawings. It will, however, be evident that various modifications and changes may be made thereto, and additional embodiments may be implemented, without departing from the scope of the invention as set forth in the claims that follow. For example, certain features of one embodiment described herein may be combined with or substituted for features of another embodiment described herein. The description and drawings are accordingly to be regarded in an illustrative rather than a restrictive sense and are not intended to be exhaustive or to limit the invention to any precise form disclosed. Many modifications and variations are possible in light of the above teaching. Thus, while the invention(s) herein disclosed has been described by means of specific embodiments and applications thereof, numerous modifications and variations could be made thereto by those skilled in the art without departing from the scope of the invention(s) set forth in the claims.

There may be provided an Implantable ElectroAcupuncture Device (IEAD) for treating a specified medical condition of a patient through application of electroacupuncture (EA) stimulation pulses applied substantially at or near a specified target tissue location, comprising: a small, thin, disc-shaped IEAD housing having an electrode configuration thereon that includes at least two electrodes/arrays, wherein the longest linear dimension of the small, thin, IEAD housing is no greater than about 25 mm, and wherein the thickness of the housing, measured in a plane orthogonal to the longest linear dimension, is no greater than 3 mm, and wherein at least one of said at least two electrodes/arrays comprises a central electrode/array located substantially in the center of a first planar surface of the IEAD housing, and wherein at least another of said at least two electrodes/arrays comprises a circumferential electrode/array located substantially around and at least 5 mm distant from the center of the central electrode/array, wherein the first surface of the IEAD housing when implanted is adapted to face inwardly into the patient's tissue at or near the specified target tissue location; pulse generation circuitry located within the IEAD housing and electrically coupled to the at least two electrodes/arrays, wherein said pulse generation circuitry is adapted to deliver stimulation pulses to the patient's body tissue at or near the target tissue location in accordance with a specified stimulation regimen, said stimulation regimen defining the duration and rate at which a stimulation session is applied to the patient, said stimulation regimen requiring that the stimulation session have a duration of T3 minutes and a rate of occurrence of once every T4 minutes, wherein the ratio of T3/T4 is no greater than 0.05, and wherein during each stimulation session EA stimulation pulses having one or more specified widths and amplitudes are generated at one or more specified rates; a thin, coin-cell, primary battery contained within the IEAD housing and electrically coupled to the pulse generation circuitry that provides operating power for the pulse generation circuitry, said primary battery having a nominal output voltage of 3 volts, and an internal impedance greater than 5 ohms; and a sensor contained within the IEAD housing responsive to operating commands wirelessly communicated to the IEAD from a non-implanted location, said operating commands allowing limited external control of the IEAD.

The small IEAD housing may be coin-shaped having a diameter no greater than about 25 mm and a thickness of no greater than about 2.5 mm.

The pulse generation circuitry may include a boost converter circuit that boosts the nominal voltage of the primary battery to an output voltage V_{OUT} that is at least three times the nominal battery voltage; means for selectively turning the boost converter circuit OFF and ON to limit the amount of current that may be drawn from the primary battery; and an output circuit powered by V_{OUT} that generates the stimulation pulses as defined by the specified stimulation regimen.

The stimulation pulses generated by the pulse generation circuit and delivered through the at least two electrodes/arrays into a load at the target tissue location may comprise current pulses having a current amplitude of no less than about 1 milliampere (mA) and no greater than about 25 mA.

The thin coin-cell primary battery may have sufficient capacity to power the pulse generation circuitry in accordance with the specified stimulation regimen for a minimum of 2 years.

The means for selectively turning the boost converter circuit OFF and ON may comprise a control circuit configured to generate a digital signal that modulates the boost converter circuit between an OFF state and an ON state, with the ON state comprising no more than about 2% of the time that the IEAD is operating.

The means for selectively turning the boost converter circuit OFF and ON may comprise a boost converter circuit having a shutdown feature which automatically places the boost converter circuit in an OFF state whenever the battery voltage falls below a set minimum value, wherein the set minimum value of the battery below which the boost converter is placed in an OFF state comprises a voltage that is sufficiently high to continue to power other digital processing circuits within the pulse generation circuitry even when the boost converter is turned OFF.

The pulse generation circuitry may further include means for reducing leading edge transient signals that may be present in the stimulation pulses generated by the pulse generation circuitry.

The output circuit of the pulse generation circuitry may include a programmable current source, and wherein the means for reducing leading edge transient signals comprises connecting a cascode circuit at the input of the programmable current source.

The pulse generation circuitry further may include means for kick starting the programmable current source when low amplitude stimulation pulses are generated, wherein such kick starting eliminates or reduces undesired delays in the leading edge of the stimulation pulses generated by the pulse generation circuitry.

The small, thin, disc-shaped IEAD housing may comprise a case having a bottom and a side wall, said case having the form of a shallow container, closed at one end and open at the other end, said case being made from a biocompatible metal; a recess formed as an integral part of the side wall at one location of the side wall, said recess extending into the space defined by the case a prescribed amount and having an opening in the bottom thereof; a feed-through pin assembly comprising a feed-through pin embedded or brazed within an insulating material, the insulating material being hermetically brazed to the opening in the bottom of the recess, with a distal end of the feed-through pin extending radially outward beyond the side wall of the case, and with a proximal end of the feed-through pin extending radially inward towards the center of the volume within the case; an electronic circuit assembly mounted inside of the case, with the proximal end of the feed-through pin being mechanically and electrically connected to a first designated output port of the electronic assembly, and with a second designated output port of the electronic assembly being connected to the case, the electronic circuit assembly including the pulse generation circuitry, and the feed-through pin assembly providing an electrical connection between the pulse generation circuitry and at least one of the at least two electrodes/arrays; a cover plate hermetically bonded to the edges of the side walls of the case, the first electrode comprising a plate centrally bonded to the bottom of the case on the outside of the case; the second electrode comprising a ring electrode positioned around the side wall and electrically connected to the distal tip of the feed-through pin at the location where the recess is formed; an insulating layer of material positioned between the ring electrode and the side wall of the case, the insulating layer preventing the second electrode from electrically contacting the case; and an insulating layer of a biocompatible non-conductive material covering all portions of the outside of the case and cover plate except for exposed surface areas of the first electrode plate and the second electrode ring.

The case and cover plate may be made from titanium, stainless steel or platinum.

The feed-through pin may be made from pure platinum or platinum iridium.
The insulating layer of non-conductive material that covers all but the second electrode and first electrode may be made from silicone molding or dip coating.

The width of the IEAD package may be no greater than 2.5 mm thick. The case and cover plate may be round.
There may be provided an implantable neurostimulation device (INSD) adapted to generate and apply electrical stimulus pulses to a target tissue location of a patient when the INSD is implanted at or near a target tissue location, the INSD comprising: an hermetically sealed case having a linear dimension D2 in first plane no greater than about 25 mm, and a linear dimension W2 in a second plane orthogonal to the first plane no greater than about 2.5 mm; at least one anodic electrode and at least one cathodic electrode secured to an outside surface of the hermetically sealed case, the separation between the point where the cathodic electrode is closest to the anodic electrode being at least 5 mm; electronic circuitry housed within the hermetically sealed case that causes electrical stimulus pulses to be generated and applied to the at least one cathodic electrode and the at least one anodic electrode in accordance with a prescribed stimulation regime: means for electrically connecting the electronic circuitry on the inside of the hermetically sealed case to the at least two electrodes on the outside of the hermetically sealed case; and a coin-cell primary battery on the inside of the hermetically sealed case connected to the electronic circuitry that provides operating power for the electronic circuitry, the primary battery having a nominal diameter no greater than about ¾ D2, a nominal output voltage of V_{BAT} volts, where V_{BAT} ranges from as low 2.2 volts to as high as 3.6 volts; wherein the coin-cell primary battery has an internal impedance greater than 5 ohms; and wherein the electronic circuitry includes power management circuitry that limits the amount of instantaneous current that can be drawn from the battery; and wherein the electronic circuitry controls the generation of the electrical stimulus pulses so that the stimulus pulses are applied only during a stimulation session having a duration of T3 minutes, and wherein the time interval between stimulation sessions is T4 minutes, and wherein the ratio of T3/T4 is no greater than 0.05.

The electronic circuitry may include: a boost converter circuit that boosts the battery voltage V_{BAT} to an output voltage V_{OUT}, where V_{OUT} is at least 3 times V_{BAT} and no more than 8 times V_{BAT}; a microcontroller circuit powered by V_{BAT} that controls the operation of the electronic circuitry so that the stimulus pulses are generated at a specified amplitude in accordance with the prescribed stimulation regime; a programmable current source circuit that sets the amplitude of the current in the stimulus pulses to a desired level; a sensor circuit adapted to receive external commands from an external control device that provide operating commands or parameters for the microcontroller circuit, the operating commands including ON/OFF commands, and the operating parameters including defining the time T3, the time T4, and the stimulus amplitude; an electronic switch, controlled by the microcontroller circuit, that switchably applies the programmed current to the at least one cathodic electrode and the at least one anodic electrode so that when the IEAD is implanted in the patient such switching is adapted to cause the programmed current to flow through the patient's body tissue at or near the specified target tissue location.

The microcontroller circuit may control the operation of the boost converter circuit in a way that limits the amount of instantaneous current that can be drawn from the battery, thereby preventing the battery voltage VBAT from dropping to values that would prevent the electronic circuitry from operating.

The microcontroller circuit may control the operation of the boost converter circuit to limit the amount of instantaneous current that can be drawn from the battery by duty-cycle modulating the boost converter circuit with a digital control signal that causes the boost converter circuit to switch between an ON state for a short time and to an OFF state for a long time, the ratio of the ON time to the OFF time being less than 0.02.

The boost converter circuit may automatically shut down whenever the battery voltage V_{BAT} drops below a fixed threshold, wherein the battery voltage below the fixed threshold is still sufficiently high to permit operation of the microcontroller circuit, and wherein the automatic shutting down of the boost converter circuit significantly decreases the current being drawn from the battery, whereby due to the high internal resistance of the battery, the battery voltage increases when the boost converter circuit shuts down and decreases when the boost converter circuit is ON, thereby causing the boost converter circuit to automatically switch between an ON state and an OFF state, which switching limits the amount of current that can be drawn from the battery.

The nominal battery voltage may be 3 volts, and the fixed threshold below which the boost converter circuit shuts down may be 2.5 volts.

The electronic circuitry may further include means for kick starting the programmable current source when the stimulus current amplitude is less than about 2 mA, which kick starting has the effect of driving the programmable current source directly from the microcontroller circuit at the beginning of the stimulus pulse, which action shortens a delay associated with starting the operation of the programmable current source at low current amplitudes by a significant factor.

The electronic circuitry may further includes a cascode circuit connected to the input terminal of the programmable current source circuit, which cascode circuit is adapted to significantly reduce the input capacitance looking into the cascode circuit over the capacitance that is seen looking into the input terminal of the programmable current source, wherein transient signals appearing at the leading edge of the stimulus pulse are significantly reduced.

The at least one cathodic electrode may comprise a central electrode located substantially in the center of a first surface of the IEAD housing, wherein the at least one anodic electrode comprises a circumferential electrode located substantially around and at least 5 mm distant from the center of the central electrode, wherein the first surface of the IEAD housing when implanted is adapted to face inwardly into the patient's tissue at or near the specified target tissue location.

The hermetically sealed case may be coin shaped, wherein the circumferential electrode comprises a ring electrode positioned around the circumferential edge of the coin-shaped case.

The means for electrically connecting the electronic circuitry on the inside of the hermetically sealed case to the at least two electrodes on the outside of the hermetically sealed case may comprise a recess formed as an integral part of a side wall of said hermetically sealed case at one location of said side wall, said recess extending into the space defined by the case a prescribed amount and having an opening in the bottom thereof; a feed-through pin assembly comprising a feed-through pin 130) embedded or brazed within an insulating material, the insulating material being hermetically brazed to the opening in the bottom of the recess, with a distal end of the feed-through pin extending radially outward beyond the side wall of the case, and with a proximal end of the feed-through pin extending radially inward towards the center of the volume within the case; wherein the distal end of the feed-through pin connects with the ring electrode positioned around the circumferential edge of the hermetically sealed case, and wherein the proximal end of the feed-through pin connects with the electronically circuitry on the inside of the hermetically sealed case.

There may be provided a method of operating an implantable electroacupuncture (EA) device powered only by a thin, coin-cell primary battery having an internal impedance greater than 5 ohms, over a period of at least 2 years, the EA device being adapted to generate stimulus pulses during a stimulation session, the method comprising: powering pulse generation circuitry within the EA device with only the coin-cell primary battery, limiting the duration of stimulation sessions during which stimulus pulses are generated to a time period that has a duty cycle of less than 0.05, where the duty cycle is the ratio of T3 to T4, where T3 is the duration of the stimulation session, and where T4 is the time interval between stimulation sessions; boosting the battery voltage from the coin-cell primary battery using a boost converter circuit by a factor of at least 4 in order to provide sufficient operating power to generate current stimulus pulses of up to 25 mA during a stimulation session; and limiting the instantaneous current that can be drawn from the coin-cell primary battery to prevent the battery voltage, V_{BAT}, from dropping below safe operating levels.

The method may comprise limiting the instantaneous current that can be drawn from the coin-cell primary battery comprises duty-cycle modulating the boost converter circuit so that it switches between an ON state and an OFF state, with the ON state having a duration of no more than 2% of the duration of the OFF state.

Duty-cycle modulating the boost converter circuit may comprise using a boost converter circuit that automatically shuts down when the battery voltage drops below a prescribed threshold, which shutting down of the boost converter circuit immediately causes the instantaneous current drawn from the battery to decrease, which decrease in instantaneous battery current causes the battery voltage to begin to increase, whereby the battery voltage is not allowed to drop below levels that would prevent the proper operation of the EA device.

There may be provided a small, thin, hermetically sealed package for housing an implantable electroacupuncture EA device, comprising: a shallow coin-sized case having a bottom plate of width D2 and a side wall connected to a perimeter edge of the bottom plate, the side wall having a height W2, wherein the ratio of W2 to D2 is no greater than about 0.12, and wherein D2 is no greater than about 25 mm; a recess formed at one portion of the side wall, said recess in the side wall extending radially inward toward the center of the bottom plate, and having a radial depth of D5 measured from the perimeter edge of the bottom plate, the side wall having an opening therein at or near the point where the recess has its maximum depth; a feed-through pin; and an insulating material through which the feed-through pin passes; wherein the insulating material is hermetically bonded to the opening in the bottom of the recess, and the surface of the pin is hermetically bonded to the insulating material, and further wherein a distal end of the feed-through pin extends radially outward for at least a distance D5 to the top of the recess, and wherein a proximal end of the feed-through pin extends radially inward towards the center of the case, whereby a feed-through assembly is created wherein electronic circuitry placed inside of the case that is connected to the proximal end of the feed-through pin provides an electrical connection with the electronic circuitry at a distal end of the feed-through pin; and a cover plate welded to the upper edge of the side wall of the case, thereby hermetically sealing the electronic circuitry inside of the case and cover, and wherein an hermetically-sealed feed-through connection is established with the electronic circuitry through the distal end of the feed-through pin.

The device may further include a circumscribing electrode connected to the distal end of the feed-through pin, the circumscribing electrode extending around the outside surface of the side wall of the case; and an insulating layer of non-conductive material positioned between the circumscribing electrode and the outside surface of the case, thereby preventing the circumscribing electrode from making electrical contact with the outside surface of the case.

The device may further include a plate electrode attached centrally to the outside of the bottom surface of the case; and a layer of non-conductive material covering all external surface areas of the case and cover except the circumscribing electrode and plate electrode.

The case and cover may be round.
There may be provided an implantable electroacupuncture device (IEAD) for treating a specified medical condition of a patient through application of electroacupuncture (EA) stimulation pulses applied substantially at or near a specified target tissue location of a patient, comprising: a coin-sized and -shaped IEAD housing having a first surface, a second surface, and an edge surface connecting the first surface to the second surface, the width of the edge surface being no greater than about 2.5 mm, wherein the volume between the first and second surfaces and bounded by the edge surface defines a space wherein components of the IEAD reside; an electrode configuration located on the outside surfaces of the IEAD housing, said electrode configuration including at least two electrodes, wherein at least one of said at least two electrodes comprises a central electrode located substantially in the center of the first surface of the IEAD housing, and wherein at least another of said at least two electrodes comprises a circumferential electrode located substantially around and at least 5 mm distant from the closest edge of the central electrode; at least one radial recess formed in the edge surface of the IEAD housing, the radial recess having a radial depth (D5) that is greater than the width of the edge surface, and an opening in the edge surface at a bottom portion of the recess, the bottom portion of the recess comprising that portion of the recess closest to the center of the IEAD housing; a feed-through pin assembly mounted so as to pass through the opening in the edge surface of the at least one radial recess, the feed-through assembly including: a feed-through pin, and an insulating material through which the feed-through pin passes, wherein the insulating material is hermetically bonded to the opening in the bottom of the recess, and the surface of the feed-through pin is hermetically bonded to the insulating material, and further wherein a distal end of the feed-through pin extends radially outward from the at least one radial recess for at least a distance D5 to the top of the recess to a point outside of the housing where the distal end is electrically connected to at least one of said at least two electrodes, and wherein a proximal end of the feed-through pin extends radially inward inside of the housing towards the center of the IEAD housing; pulse generation circuitry located within the IEAD housing and electrically coupled to a proximal end of the feed-through pin extending inwardly into the housing from the at least one radial recess, wherein said pulse generation circuitry is adapted to deliver stimulation pulses to the patient's body tissue through the at least two electrodes located on the surface of the IEAD housing in accordance with a specified stimulation regimen, said stimulation regimen defining the duration and rate at which a stimulation session is applied to the patient, said stimulation regimen requiring that the stimulation session has a duration of T3 minutes and a rate of occurrence of once every T4 minutes, wherein the ratio of T3/T4 is no greater than 0.05, and wherein during each stimulation session EA stimulation pulses having one or more specified widths and amplitudes are generated at one or more specified rates; a thin, coin-cell primary battery contained within the IEAD housing and electrically coupled to the pulse generation circuitry that provides operating power for the pulse generation circuitry, said primary battery having a nominal output voltage of 3 volts, and an internal impedance greater than 5 ohms; and a sensor contained within the IEAD housing responsive to operating commands wirelessly communicated to the IEAD from a non-implanted location, said operating commands allowing limited external control of the IEAD.

The surfaces of the IEAD housing may be made from a biocompatible material that is electrically conductive, and wherein the central electrode comprises a plate centrally bonded to the outside of the first surface of the housing so as to be in electrical contact therewith; and further wherein the circumferential electrode comprises a ring electrode positioned around the edge surface, an underneath side of the circumferential electrode being electrically connected to the distal tip of the feed-through pin at the location where the at least one radial recess is formed; and wherein the IEAD housing further includes: an insulating layer of material positioned between the underneath side of the circumferential electrode and the side wall of the housing, the insulating layer preventing the circumferential electrode from electrically contacting the case; and an insulating layer of a biocompatible non-conductive material covering all portions of the outside of the housing except for outward facing surface areas of the central electrode and the circumferential electrode.

There may be provided a method of assembling an implantable electroacupuncture (EA) device in a small, thin, hermetically sealed, housing having a maximum linear dimension D2 in a first plane of no more than 25 mm and a maximum linear dimension W2 in a second plane orthogonal to the first plane of no more than 2.5 mm, the housing having at least one feed-through pin assembly therein that radially passes through a wall of the thin housing, the method being adapted to isolate the feed-through pin assembly from high temperatures and residual weld stresses that it would otherwise be subjected to when the thin housing is welded shut to hermetically-seal its contents, the method of assembly comprising: forming a thin housing having a bottom case and a top cover plate, the top cover plate being adapted to fit over the bottom case, the bottom case having a maximum linear dimension of no more than 25 mm; forming a recess in a wall of the housing; placing a feed-through assembly within the recess so that a feed-through pin of the feed-through assembly electrically passes through a wall of the recess at a location that is separated from where the wall of the housing is designed to contact the top cover plate; and welding the top cover plate to the bottom case around a perimeter of the bottom case, thereby hermetically sealing the bottom case and cover plate together.

There may be provided an Implantable ElectroAcupuncture Device (IEAD) adapted for implantation in a patient and for treating a specified medical condition of the patient through application of electroacupuncture (EA) stimulation pulses applied substantially at or near a specified target tissue location of the patient, comprising: a small, thin, disc-shaped IEAD housing having an electrode configuration thereon that includes at least two electrodes/arrays, the diameter of the disc-shaped IEAD housing being no greater than about 25 mm, and the thickness of the disc-shaped housing being no greater than about 2.5 mm, wherein at least one of said at least two electrodes/arrays comprises a central electrode/array located substantially in the center of a first surface of the IEAD housing, and wherein at least another of said at least two electrodes/arrays comprises a circumferential electrode/array located substantially around and at least 5 mm distant from the center of the central electrode/array, wherein the first surface of the IEAD housing when implanted is adapted to face towards the specified target tissue location of the patient; pulse generation circuitry located within the disc-shaped IEAD housing and electrically coupled to the at least two electrodes/arrays, wherein said pulse generation circuitry is adapted to deliver stimulation pulses to the patient's body tissue at or near the target tissue location in accordance with a specified stimulation regimen, said stimulation regimen defining the duration and rate at which a stimulation session is applied to the patient, said stimulation regimen requiring that the stimulation session have a duration of T3 minutes and a rate of occurrence of once every T4 minutes, wherein the ratio of T3/T4 is no greater than 0.05, and wherein during each stimulation session EA stimulation pulses having one or more specified widths and amplitudes are generated at one or more specified rates; a coin-cell primary battery contained within the disc-shaped IEAD housing and electrically coupled to the pulse generation circuitry that provides operating power for the pulse generation circuitry, said primary battery having a nominal output voltage of 3 volts, and an internal impedance greater than 5 ohms; and a sensor contained within the IEAD housing responsive to operating commands wirelessly communicated to the IEAD from a non-implanted location, said operating commands allowing limited external control of the IEAD.

The pulse generation circuitry may include: a boost converter circuit that boosts the nominal voltage of the primary battery to an output voltage V_{OUT} that is at least three times the nominal battery voltage; means for selectively turning the boost converter circuit OFF and ON to limit the amount of current that may be drawn from the primary battery; and an output circuit powered by V_{OUT} that generates the stimulation pulses as defined by the specified stimulation regimen having a current amplitude of up to about 25 mA.

The specified medical condition may comprise at least one of (1) hypertension, (2) cardiovascular disease, (3) depression, (4) bipolar disorder, (5) Anxiety, (6) obesity, (7) dyslipidemia, (8) Parkinson's disease, (9) Essential tremor, or (10) erectile dysfunction.

The specified target tissue location may comprise at least one acupoint selected from the group of acupoints comprising: BL14, BL23, BL52, EXHN1, EXHN3, GB34, GV4, GV20, HT5, HT7, KI6, LI4, LI11, LR3, LR8, LU2, LU7, PC5, PC6, PC7, SP4, SP6, SP9, ST36, ST37, and ST40.

The specified medical condition may comprise hypertension and wherein the specified target tissue location comprises at least one of the acupoints PC5, PC6, ST36 or ST37.

The specified medical condition may comprise at least one of depression, bipolar disorder and Anxiety, and wherein the specified target tissue location comprises at least one of the acupoints GV20 or EXHN3.

The specified medical condition may comprise cardiovascular disease and wherein the specified target tissue location comprises at least one of the acupoints: BL14, EXHN1, HT5, HT7, LI11, LU2, LU7, PC6, or ST36.

The specified medical condition may comprise dyslipidemia or obesity and wherein the specified target tissue location comprises at least one of the acupoints: KI6, LR8, SP4, SP6, SP9, ST36, ST37 or ST40.

The specified medical condition may comprise Parkinson's disease or Essential tremor and wherein the specified target tissue location comprises at least one of the acupoints GB34 or GV20.

The specified medical condition may comprise erectile dysfunction and wherein the specified target tissue location comprises at least one of the acupoints GV4, BL23 or BL52.

There may be provided method of treating a disease or medical condition of a patient using a thin, disc-shaped implantable electroacupuncture device (IEAD) powered only by a thin, coin-cell type battery having an internal impedance greater than 5 ohms, over a period of at least 2 years, the IEAD having at least two electrodes formed as an integral part of its housing, and being adapted to generate stimulus pulses that are applied to its electrodes during a stimulation session, the method comprising: implanting the IEAD below the skin surface of a patient at or near a specified target tissue location; powering pulse generation circuitry within the IEAD with the coin-cell type battery; limiting the duration of stimulation sessions during which stimulus pulses are generated to a time period that has a duty cycle of less than 0.05, where the duty cycle is the ratio of T3 to T4, where T3 is the duration of the stimulation session, and where T4 is the time interval between stimulation sessions; boosting the battery voltage from the coin-cell type battery using a boost converter circuit by a factor of at least 4 in order to provide sufficient operating power to generate current stimulus pulses of up to 25 mA during a stimulation session; and limiting the instantaneous current that can be drawn from the coin-cell type battery to prevent the battery voltage, V_{BAT}, from dropping below safe operating levels.

## Claims

1. An implantable electroacupuncture device, comprising:
a disc-shaped housing (122,124);
pulse generation circuitry (133) located within the housing, wherein the pulse generation circuitry is adapted to
generate stimulation sessions at a duty cycle that is less than 0.05, and
apply the stimulation sessions to a location within a patient by way of an electrode array (110, 120, 310, 320,E1, E2) communicatively coupled to the pulse generation circuitry; and
a primary battery (132, 215) located within the housing and having an internal impedance greater than 5 ohms, the primary battery configured to provide operating power to the pulse generation circuitry;
wherein
each stimulation session included in the stimulation sessions comprises a series of stimulation pulses,
the duty cycle is a ratio of T3 to T4, and
each stimulation session included in the stimulation sessions has a duration of T3 minutes and occurs at a rate of once every T4 minutes.

2. The implantable electroacupuncture device of claim 1, wherein the primary battery (132, 215) located within the housing has a capacity of less than 60 milliamp-hours (mAh).

3. The implantable electroacupuncture device of claim 1, wherein:
the housing (122, 124) of the electroacupuncture device (100) is coin-sized and coin-shaped; and
the primary battery (132, 215) located within the housing is a coin-cell battery.

4. The implantable electroacupuncture device of claim 1, wherein:
the electrode array (110, 120, 310, 320,E1, E2) comprises a central electrode (110, 310) of a first polarity centrally located on a first surface of the housing (122, 124) of the electroacupuncture device (100) and an annular electrode (120, 320) of a second polarity and that is spaced apart from the central electrode; and
the pulse generation circuitry (133) is configured to apply the stimulation sessions to the location by way of the electrode array by applying the stimulation sessions to the location by way of the central electrode and the annular electrode.

5. The implantable electroacupuncture device of claim 4, wherein the annular electrode (120, 320) is located on the first surface of the housing (122, 124).

6. The implantable electroacupuncture device of claim 4, wherein the annular electrode comprises a ring electrode (120) located around a perimeter edge of the housing.

7. The implantable electroacupuncture device of claim 1, wherein T3 is at least 10 minutes and less than 60 minutes, and wherein T4 is at least 1440 minutes.

8. An implantable electroacupuncture device, comprising:
a disc-shaped housing (122, 124);
pulse generation circuitry (133) located within the housing, wherein the pulse generation circuitry is adapted to
generate stimulation sessions at a duty cycle that is less than 0.05, and
apply the stimulation sessions to a location within a patient by way of an electrode array (110, 120, 310, 320,E1, E2) communicatively coupled to the pulse generation circuitry; and
a primary battery (132, 215) located within the housing and having a capacity of less than 60 milliamp-hours (mAh), the primary battery configured to provide operating power to the pulse generation circuitry;
wherein
each stimulation session included in the stimulation sessions comprises a series of stimulation pulses,
the duty cycle is a ratio of T3 to T4, and
each stimulation session included in the stimulation sessions has a duration of T3 minutes and occurs at a rate of once every T4 minutes.

9. The implantable electroacupuncture device of claim 8, wherein the primary battery (132, 215) located within the housing (122, 124) has an internal impedance greater than 5 ohms.

10. The implantable electroacupuncture device of claim 8, wherein:
the housing (122, 124) of the electroacupuncture device (100) is coin-sized and coin-shaped; and
the primary battery (132, 215) located within the housing is a coin-cell battery.

11. The implantable electroacupuncture device of claim 8, wherein T3 is at least 10 minutes and less than 60 minutes, and wherein T4 is at least 1440 minutes.

## Patentansprüche

1. Implantierbares Elektroakkupunkturgerät mit
einem scheibenförmigen Gehäuse (122, 124);
einer Pulserzeugungsschaltung (133), die in dem Gehäuse angeordnet ist und ausgebildet ist, um:
Stimulationssitzungen bei einem Tastverhältnis von weniger als 0,05 zu erzeugen, und
die Stimulationssitzungen an einer Stelle eines Patienten mittels einer Elektrodenanordnung (110, 120, 310, 320, E1, E2) anzuwenden, die kommunikativ mit der Pulserzeugungsschaltung gekoppelt ist; und
einer Primärbatterie (132, 215), die in dem Gehäuse angeordnet ist und eine innere Impedanz von mehr als 5 Ω aufweist und ausgebildet ist, um die Pulserzeugungsschaltung mit Strom zu versorgen;
wobei
jede Stimulationssitzung der Stimulationssitzungen eine Serie von Stimulationspulsen beinhaltet,
es sich bei dem Tastverhältnis um ein Verhältnis von T3 zu T4 handelt, und
jede Stimulationssitzung der Stimulationssitzungen eine Dauer von T3 Minuten aufweist und mit einer Rate von einmal alle T4 Minuten auftritt.

2. Implantierbares Elektroakkupunkturgerät gemäß Anspruch 1, wobei die Primärbatterie (132, 215), die in dem Gehäuse angeordnet ist, eine Kapazität von weniger als 60 Milliamperstunden (mAh) aufweist.

3. Implantierbares Elektroakkupunkturgerät gemäß Anspruch 1, wobei:
das Gehäuse (122, 124) des Elektroakkupunkturgeräts (100) die Größe einer Münze aufweist und münzenförmig ausgebildet ist; und
die Primärbatterie (132, 215), die in dem Gehäuse angeordnet ist, eine Knopfzellenbatterie ist.

4. Implantierbares Elektroakkupunkturgerät gemäß Anspruch 1, wobei:
die Elektrodenanordnung (110, 120, 310, 320, E1, E2) eine zentrale Elektrode (110, 310) mit einer ersten Polarität, die zentral auf einer ersten Oberfläche des Gehäuses (122, 124) des Elektroakkupunkturgeräts (100) angeordnet ist, sowie eine ringförmige Elektrode (120, 320) mit einer zweiten Polarität, die in Abstand von der zentralen Elektrode angeordnet ist, aufweist; und
die Pulserzeugungsschaltung (133) ausgebildet ist, um die Stimulationssitzungen an der Stelle mittels der Elektrodenanordnung anzuwenden, indem die Stimulationssitzungen an der Stelle mittels der zentralen Elektrode und der ringförmige Elektrode angewandt werden.

5. Implantierbares Elektroakkupunkturgerät gemäß Anspruch 4, wobei die ringförmige Elektrode (120, 320) auf einer ersten Oberfläche des Gehäuses (122, 124) angeordnet ist.

6. Implantierbares Elektroakkupunkturgerät gemäß Anspruch 4, wobei die ringförmige Elektrode eine Ringelektrode (120) aufweist, die um einen Umfangsrand des Gehäuses herum angeordnet ist.

7. Implantierbares Elektroakkupunkturgerät gemäß Anspruch 1, wobei T3 mindestens 10 Minuten und weniger als 60 Minuten beträgt und wobei T4 mindestens 1440 Minuten beträgt.

8. Implantierbares Elektroakkupunkturgerät mit:
einem scheibenförmigen Gehäuse (122, 124);
einer Pulserzeugungsschaltung (133), die innerhalb des Gehäuses angeordnet ist und ausgebildet ist, um
Stimulationssitzungen mit einem Tastverhältnis von weniger als 0,05 zu erzeugen, und
die Stimulationssitzungen an einer Stelle innerhalb eines Patienten mittels einer Elektrodenanordnung (110, 120, 310, 320, E1, E2) anzuwenden, die kommunikativ mit der Pulserzeugungsschaltung gekoppelt ist; und
einer Primärbatterie (132, 215), die innerhalb des Gehäuses angeordnet ist und eine Kapazität von weniger als 60 Milliamperestunden (mAh) aufweist und ausgebildet ist, um die Pulserzeugungsschaltung mit Strom zu versorgen;
wobei
jede Stimulationssitzung der Stimulationssitzungen eine Serie von Stimulationspulsen aufweist,
es sich bei dem Tastverhältnis um ein Verhältnis von T3 zu T4 handelt, und
jede Stimulationssitzung der Stimulationssitzungen eine Dauer von T3 Minuten aufweist und bei einer Rate von einmal alle T4 Minuten auftritt.

9. Implantierbares Elektroakkupunkturgerät gemäß Anspruch 8, wobei die Primärbatterie (132, 215), die innerhalb des Gehäuses (122, 124) angeordnet ist, eine innere Impedanz von mehr als 5 Ω aufweist.

10. Implantierbares Elektroakkupunkturgerät gemäß Anspruch 8, wobei:
das Gehäuse (122, 124) des Elektroakkupunkturgeräts (100) die Abmessungen einer Münze aufweist und münzenförmig ausgebildet ist; und
die Primärbatterie (132, 215), die innerhalb des Gehäuses angeordnet ist, eine Knopfzellenbatterie ist.

11. Implantierbares Elektroakkupunkturgerät gemäß Anspruch 8, wobei T3 mindestens 10 Minuten und weniger als 60 Minuten ist und wobei T4 mindestens 1440 Minuten ist.

## Revendications

1. Dispositif d'électro-acupuncture implantable, comprenant :
un boîtier en forme de disque (122, 124) ;
des circuits de génération d'impulsion (133) situés à l'intérieur du boîtier, les circuits de génération d'impulsion étant adaptés pour
générer des sessions de stimulation avec un cycle opératoire qui est inférieur à 0,05, et
appliquer les sessions de stimulation à un site chez un patient au moyen d'un réseau d'électrodes (110, 120, 310, 320, E1, E2) couplées en communication aux circuits de génération d'impulsion ; et
une batterie primaire (132, 215) située à l'intérieur du boîtier et ayant une impédance interne supérieure à 5 ohms, la batterie primaire étant configurée pour fournir une puissance opérationnelle aux circuits de génération d'impulsion ;
dans lequel
chaque session de stimulation comprise dans les sessions de stimulation comprend une série d'impulsions de stimulation,
le cycle opératoire est un rapport de T3 à T4, et
chaque session de stimulation comprise dans les sessions de stimulation présente une durée de T3 minutes et survient avec une fréquence d'une fois toutes les T4 minutes.

2. Dispositif d'électro-acupuncture implantable selon la revendication 1, dans lequel la batterie primaire (132, 215) située à l'intérieur du boîtier présente une capacité de moins de 60 milliampères-heure (mAh).

3. Dispositif d'électro-acupuncture implantable selon la revendication 1, dans lequel :
le boîtier (122, 124) du dispositif d'électro-acupuncture (100) est de la taille et de la forme d'une pièce de monnaie ; et
la batterie primaire (132, 215) située à l'intérieur du boîtier est une pile bouton.

4. Dispositif d'électro-acupuncture implantable selon la revendication 1, dans lequel :
le réseau d'électrodes (110, 120, 310, 320, E1, E2) comprend une électrode centrale (110, 310) d'une première polarité située de manière centrale sur une première surface du boîtier (122, 124) du dispositif d'électro-acupuncture (100) et une électrode annulaire (120, 320) d'une deuxième polarité et qui est espacée de l'électrode centrale ; et
les circuits de génération d'impulsion (133) sont configurés pour appliquer les sessions de stimulation au site au moyen du réseau d'électrodes en appliquant les sessions de stimulation au site au moyen de l'électrode centrale et de l'électrode annulaire.

5. Dispositif d'électro-acupuncture implantable selon la revendication 4, dans lequel l'électrode annulaire (120, 320) est située sur la première surface du boîtier (122, 124).

6. Dispositif d'électro-acupuncture implantable selon la revendication 4, dans lequel l'électrode annulaire comprend une électrode circulaire (120) située autour d'un pourtour du boîtier.

7. Dispositif d'électro-acupuncture implantable selon la revendication 1, dans lequel T3 correspond à au moins 10 minutes et moins de 60 minutes, et T4 correspond à au moins 1440 minutes.

8. Dispositif d'électro-acupuncture implantable, comprenant :
un boîtier en forme de disque (122, 124) ;
des circuits de génération d'impulsion (133) situés à l'intérieur du boîtier, les circuits de génération d'impulsion étant adaptés pour
générer des sessions de stimulation avec un cycle opératoire qui est inférieur à 0,05, et
appliquer les sessions de stimulation à un site chez un patient au moyen d'un réseau d'électrodes (110, 120, 310, 320, E1, E2) couplées en communication aux circuits de génération d'impulsion ; et
une batterie primaire (132, 215) située à l'intérieur du boîtier et ayant une capacité inférieure à 60 milliampères-heure (mAh), la batterie primaire étant configurée pour fournir de l'énergie opérationnelle aux circuits de génération d'impulsion ;
dans lequel
chaque session de stimulation comprise dans les sessions de stimulation comprend une série d'impulsions de stimulation,
le cycle opératoire est un rapport de T3 à T4, et
chaque session de stimulation comprise dans les sessions de stimulation présente une durée de T3 minutes et survient à une fréquence d'une fois toutes les T4 minutes.

9. Dispositif d'électro-acupuncture implantable selon la revendication 8, dans lequel la batterie primaire (132, 215) située à l'intérieur du boîtier (122, 124) présente une impédance interne supérieure à 5 ohms.

10. Dispositif d'électro-acupuncture implantable selon la revendication 8, dans lequel :
le boîtier (122, 124) du dispositif d'électro-acupuncture (100) est de la taille et de la forme d'une pièce de monnaie ; et
la batterie primaire (132, 215) située à l'intérieur du boîtier est une pile bouton.

11. Dispositif d'électro-acupuncture implantable selon la revendication 8, dans lequel T3 correspond à au moins 10 minutes et moins de 60 minutes, et T4 correspond à au moins 1440 minutes.
